(19) Europäisches Patentamt
European Patent Office
Office européen des brevets

(11) EP 4 632 367 A1

(12) **EUROPEAN PATENT APPLICATION**

(43) Date of publication:
**15.10.2025 Bulletin 2025/42**

(21) Application number: **24382358.0**

(22) Date of filing: **08.04.2024**

(51) International Patent Classification (IPC):
***G01N 27/49*** *(2006.01)* *G01N 27/333* *(2006.01)*

(52) Cooperative Patent Classification (CPC):
**G01N 27/49; G01N 33/492;** G01N 27/333

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **CreatSens Health S.L.**
**43204 Reus Tarragona (ES)**

(72) Inventors:
- **ZUAZNABAR GARDONA, Julio César**
  **43204 Reus (ES)**
- **GUINOVART PAVÓN, Tomàs de Aquino**
  **43204 Reus (ES)**
- **NOVELL RECASENS, Marta**
  **43204 Reus (ES)**
- **MACEIRA SOLÉ, Adrià**
  **43204 Reus (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

(54) **PHOSPHATE SENSOR AND METHOD THEREOF**

(57) The present invention refers to a phosphate sensor and a method thereof for determining the presence and/or concentration of phosphate in an isolated blood, serum and/or plasma sample by measuring the current or the voltage between a first and a second electrode, wherein at least part of the first electrode comprises a metal from the list consisting of cobalt, nickel, tungsten, iron or any of their alloys, preferably comprises cobalt.

EP 4 632 367 A1

## Description

### Technical field of the invention

The present invention belongs to the field of electrochemical sensing or electrochemical detection, particularly phosphate detection. More particularly, the present invention relates to a device and a method of phosphate detection using at least one electrode comprising cobalt, nickel, tungsten, iron or any of their alloys.

### Background of the invention

Phosphate concentration in blood plays a crucial role in maintaining the balance of minerals and overall physiological functions within the body. Abnormal phosphate levels, particularly elevated levels, known as hyperphosphatemia, are commonly observed in patients with chronic renal disease. This condition may contribute to various complications, including mineral and bone disorders, cardiovascular problems, and the progression of kidney disease.

Monitoring phosphate levels in clinical settings is typically performed through blood tests, specifically measuring blood, serum and/or plasma phosphate levels. Commonly used tests involve using various techniques such as colorimetry or enzymatic assays, which have the inconvenience of requiring a relatively long time to obtain the results, leading to delays in treatment decisions. Additionally, as these tests may require externalization to a specialised laboratory or healthcare facility, they are often expensive.

It is known that cobalt electrodes can be used in an electrochemical sensor to detect the concentration of phosphate ions in soil or water via potentiometry. These sensors are based on a direct redox reaction between the electrodes and the soil or water samples. Since cobalt oxidation is affected by the presence of phosphate ions, this results in a measurable change of the open circuit potential of the system that can be related to phosphate levels in the sample. This, in turn, yields a quick, simple and cost-effective method for assessing phosphate concentration in soil or water samples.

However, this approach is not suitable for detecting phosphate in human blood, serum and/or plasma samples due to the adsorption of proteins in the surface of said electrodes, which hinders the interaction of phosphate ions with the cobalt surface, preventing it from oxidizing and in turn negatively affecting the potentiometric response.

Other phosphate sensors capable of measuring phosphate concentration in human blood, serum and/or plasma may involve the use of complex electrodes that require nanostructures such as graphene, they may require treatment of the sample by a professional before measuring, such as diluting it or adding enzymes or reactants, or they may involve complex physical processes that require elaborate and micro-structured electrodes.

There is, therefore, the need for a phosphate sensor for undiluted, unmodified human blood, serum and/or plasma samples with a phosphate sensitivity in the human range, without the need to manufacture complex electrodes or nanostructures. This sensor should be capable of directly interact with the phosphate in blood, serum and/or plasma samples, avoiding, thus, the use of enzymes or other intermediate agents that adds complexity to the device. And, finally, there is a need for a phosphate sensor with electrodes based on simple, available, and easy to fabricate materials.

### Summary of the invention

In a first aspect of the invention, a method for determining the presence and/or concentration of phosphate in an isolated blood, serum and/or plasma sample is disclosed, said method comprising:

a. providing the isolated blood, serum and/or plasma sample in a sampling area, the sampling area comprising at least part of the surface of a first electrode, preferably the sampling area also comprising at least part of the surface of a second electrode,
and the method further comprising either one of the following steps:

b. applying a voltage between the first electrode and a second electrode and measuring a current between said first and second electrodes, or,

c. measuring a voltage between the first electrode and the second electrode,

wherein the presence and/or concentration of phosphate in the isolated blood, serum and/or plasma sample is determined by the current or the voltage measured between the first electrode and the second electrode, wherein at least the first electrode is characterized by comprising a metal from the list consisting of cobalt, nickel, tungsten, iron or any of their alloys, preferably cobalt, and at least part of the first electrode is further configured to generate a current associated to a direct redox reaction between said at least part of the first electrode and the provided isolated blood, serum and/or plasma

sample, and wherein if step c is performed, prior to said step of measuring the voltage between the first electrode and the second electrode, the pH of the isolated blood, serum and/or plasma sample is reduced below 7.4, preferably between 1 and 7, more preferably between 3.4 and 5.4.

In a preferred embodiment of the method of the first aspect of the invention for determining the presence and/or concentration of phosphate in an isolated blood, serum and/or plasma sample, the method further involves a reference electrode (14), wherein the voltage of step (b) is applied between the first electrode (10) and the reference electrode (14), and the voltage of step (c) is measured between the first electrode (10) and the reference electrode (14).

In a preferred embodiment of the method of the first aspect of the invention for determining the presence and/or concentration of phosphate in an isolated blood, serum and/or plasma sample above disclosed, the method comprises the steps of:

a. providing the isolated blood, serum and/or plasma sample in a sampling area, the sampling area comprising at least part of the surface of a first electrode,

b. applying a voltage between the first electrode and a second electrode, or between the first electrode and the reference electrode, and

c. subsequently or simultaneously to step b, measuring a current between the first electrode and the second electrode,

wherein the presence and/or concentration of phosphate in the isolated blood, serum and/or plasma sample is determined by the measured current between the first electrode and the second electrode, and wherein at least the first electrode is characterized by comprising a metal from the list consisting of cobalt, nickel, tungsten, iron or any of their alloys, preferably cobalt, and at least part of the first electrode is further configured to generate a current associated to a direct redox reaction between said at least part of the first electrode and the provided isolated blood, serum and/or plasma sample.

In a preferred embodiment of the method of the invention, at least part of the surface of the first electrode comprises a permselective membrane that allows the flow through said membrane of at least phosphate ions, wherein said permselective membrane preferably blocks the flow through said membrane of at least a reducing agent, an oxidizing agent, inorganic and/or organic species, a protein and/or a biomolecule.

According to another preferred embodiment of the method of the invention, the determination of the presence and/or concentration of phosphate in the isolated blood, serum and/or plasma sample is performed by comparing the value of the current measured with reference values of current versus phosphate concentration, preferably the reference values are obtained from a dependency curve between the current and the concentration of phosphate.

In a preferred embodiment of the method of the invention, the measured current between the first electrode and the second electrode under applied voltage is measured via amperometry, preferably chronoamperometry, or via voltammetry, preferably Linear-Sweep Voltammetry, Cyclic Voltammetry, Normal Pulse Voltammetry, Square-Wave Voltammetry or Differential Pulse Voltammetry, more preferably via Linear-Sweep Voltammetry or Cyclic Voltammetry.

In a second aspect of the invention, a phosphate sensor for determining the presence and/or concentration of phosphate in a blood, serum and/or plasma sample is disclosed, the phosphate sensor comprising a first and a second electrodes, preferably further comprising a reference electrode (14), and wherein the phosphate sensor further comprises a sampling area, said sampling area comprising at least part of the surface of a first electrode, wherein said first electrode is configured to be at least partially in contact with the blood, serum and/or plasma sample provided in the sampling area, wherein said first electrode is further configured to generate a current associated to a direct redox reaction between said at least part of the first electrode and the provided isolated blood, serum and/or plasma sample, and wherein at least the first electrode comprises a metal from the list consisting of cobalt, nickel, tungsten, iron or their alloys, preferably cobalt.

In a preferred embodiment of the second aspect of the invention, the phosphate sensor preferably further comprises a reference electrode (14), and said phosphate sensor further comprises a control unit configured to apply a voltage between the first electrode and a second electrode, or between the first electrode (10) and the reference electrode (14).

According to a more preferred embodiment of the second aspect of the invention, the phosphate sensor further comprises an analysing unit configured to measure and analyse a direct redox current generated by the voltage applied between the first electrode and the second electrode, and/or between a reference electrode and the second electrode, wherein said direct redox current is originated at the interface between the first electrode and/or the second electrode, and the blood, serum and/or plasma sample.

In another preferred embodiment of the second aspect of the invention, at least part of the surface of the first electrode of the phosphate sensor comprises a permselective membrane that allows the flow through said membrane of at least phosphate ions, wherein said permselective membrane preferably blocks the flow through said membrane of at least a reducing agent, an oxidizing agent, inorganic and/or organic species, a protein and/or a biomolecule.

According to a preferred embodiment of the second aspect of the invention, the sampling area of the phosphate

sensor further comprises at least part of the surface of a reference electrode.

In a preferred embodiment of the second aspect of the invention, the control unit of the phosphate sensor is configured to apply a constant voltage, preferably at a voltage between -2.0 and +2.0 volts, more preferably between -1.2 and +1.2 volts, even more preferably between -0.6 and +0.4 volts.

In another preferred embodiment of the second aspect of the invention, the control unit of the phosphate sensor is configured to apply a varying voltage with time, preferably varied between voltages comprised in the range between -2.0 and +2.0 volts, more preferably -1.2 and +1.2 volts, even more preferably between -0.6 and +0.4 volts.

According to a preferred embodiment of the second aspect of the invention, the analysing unit is further configured to measure current responses generated by the applied voltage via amperometry, preferably chronoamperometry, or voltammetry, preferably via Linear-Sweep Voltammetry, Cyclic Voltammetry, Normal Pulse Voltammetry, Square-Wave Voltammetry or Differential Pulse Voltammetry, more preferably via Linear-Sweep Voltammetry or Cyclic Voltammetry.

In a preferred embodiment of the second aspect of the invention, the detection range of the phosphate sensor includes the normal values of phosphate concentration in serum, plasma and/or blood of humans, preferably the detection range comprises values between 0.01 to 25 mmol/L, more preferably between 0.2 to 10 mmol/L, even more preferably between 0.3 to 3 mmol/L.

In another preferred embodiment of the second aspect of the invention, at least the first electrode of the phosphate sensor is screen printed, electrodeposited or comprises a piece of bulk metal, preferably the piece of bulk metal has the shape of a wire, a sheet, a plate, a disc or any other adequate bulk metal shape.

## Brief description of the drawings

To enable a better understanding of the present disclosure, and to show how the present disclosure may be carried out, reference will now be made, by way of example only, to the accompanying schematic drawings, wherein:

Figure 1 shows a lateral view of a schematic representation of an electrochemical sensor according to one or more embodiments of the invention.

Figure 2 shows a perspective view of a schematic representation of a screen-printed electrochemical sensor according to one or more embodiments of the invention.

Figure 3 shows a graph of the current response at different applied potentials of cobalt wire electrodes in artificial serum containing BSA after successive additions of phosphate ions, according to one embodiment of the invention.

Figure 4 shows a calibration plot obtained by Linear Sweep Voltammetry in human plasma using electrodeposited cobalt on screen-printed electrodes, according to one embodiment of the invention. The inset shows a prediction of phosphate concentrations using the cobalt-based electrodes.

Figure 5 shows a graph of the response toward phosphate ions in artificial serum containing BSA of screen-printed electrodes fabricated with different ink containing cobalt powder, according to one or more embodiments of the invention.

Figure 6 shows a calibration plot obtained by Linear Sweep Voltammetry in artificial serum containing BSA using electrodes that comprise an iron and cobalt alloy in their composition, according to one embodiment of the invention.

Figure 7 shows a calibration plot obtained by Linear Sweep Voltammetry in artificial serum containing BSA using electrodeposited nickel on screen-printed electrodes, according to one embodiment of the invention.

Figure 8 shows a calibration plot obtained by Cyclic Voltammetry in artificial serum containing BSA using electrodes that comprise tungsten in their composition, according to one embodiment of the invention.

Figure 9 shows an interpolation of three human plasma samples in a calibration curve obtained using cobalt electrodes in a screen-printed sensor according to one embodiment of the invention.

Figure 10 shows a comparison of $H_2PO_4^-$ determination (mM) in spiked human plasma samples obtained by a Co-based potentiometric sensor according to one embodiment of the invention, and the colorimetric assay.

## Description of the invention

**Definitions**

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

It is noted that the term "about", as used herein, refers to +/- 30%, preferably +/- 20%, preferably +/- 15%, more preferably +/- 10%, of the indicated referred value.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of does not exclude materials or steps that do not materially affect the basic and novel characteristics of the claim.

The term "phosphate", in the context of the invention, refers to $PO_4^{3-}$ and/or $HPO_4^{2-}$ and/or $H_2PO_4^-$ ions and/or any molecule comprising a phosphate group in their composition, that is, a functional group characterized by a phosphorus atom bonded to four oxygen atoms. Thus, the term may refer to inorganic phosphate, commonly referred as Pi, organic phosphate, such as adenosine triphosphate (ATP) or adenosine diphosphate (ADP), as well as phosphate complexes, or any combination thereof.

The term "serum", in the context of the invention, encompasses serum obtained from human blood samples and also artificial serum that imitates human serum such as, for instance, artificial serum depleted or repleted with bovine serum albumin (BSA).

The term "plasma", in the context of the invention, encompass plasma obtained from human blood samples and also artificial plasma that imitates human plasma such as, for instance, artificial plasma depleted or repleted with bovine serum albumin (BSA).

It is noted that serum and plasma primarily consist of the liquid portion of blood, along with various dissolved substances like electrolytes, proteins, hormones, and waste products, but they do not contain the cellular components of blood, including red blood cells, white blood cells, or platelets.

The primary difference between serum and plasma is that plasma is the liquid component of blood that still contains clotting factors, while serum is the liquid portion of blood that remains after clotting, and it lacks clotting factors.

The term "sampling area", in the context of the invention refers to the surface, or volume comprising said surface, intended for receiving the sample. When the sample is deposited in the "sampling area" it gets in direct contact with the electrodes of the sensor, therefore said sampling area, or volume comprising said sampling area, comprises at least part of said electrodes.

The term "working electrode" refers to the electrode where the electrochemical reaction of interest takes place. In the context of the present invention, at the surface of the "working electrode" takes place the redox reaction between the electrode and the sample. The "working electrode" may also be known as the "indicator electrode".

The term "counter electrode", also known as the auxiliary electrode, refers to the electrode that completes the electrical circuit within the cell.

The term "reference electrode" refers to an electrode that establishes a stable and known potential, serving as a reference for the measurements. In amperometry and voltammetry, a reference electrode may be used along with a working electrode and a counter electrode, in potentiometry, a reference electrode may be used along with an indicator electrode.

The term "direct redox reaction" refers to an oxidation and/or reduction reaction that involves a transfer of electrons between two species without any intermediate species involved.

The term "permselective membrane" is a membrane that selectively allows the passage of specific molecules while restricting the passage of others. In the context of the invention, a "permselective membrane" preferably allows or blocks the passage of phosphate molecules, while preferably blocking or allowing the passage of at least one reducing agent, oxidizing agent, inorganic and/or organic species, protein and/or biomolecule.

The term "oxidizing agent" refers to a chemical species that causes the oxidation of another species by accepting electrons from them.

The term "reducing agent" refers to a chemical species that causes the reduction of another species by providing electrons to them.

The term "biomolecule" refers to any organic molecule that is naturally produced within living organisms or is involved in their biological processes.

The term "amperometry" refers to a technique used in electrochemistry to measure current flow resulting from an electrochemical reaction. It involves applying a constant potential and measuring the resulting current, providing information about the concentration of analytes or the kinetics of electrochemical processes.

The term "Chronoamperometry" refers to an electrochemical technique that measures the current response over time at a fixed applied potential.

The term "voltammetry" refers to a technique used in electrochemistry to measure current flow as a function of an applied potential, resulting from an electrochemical reaction. It involves applying a varying potential and measuring the resulting current, providing information about the concentration of analytes or the kinetics of electrochemical processes

The term "Linear Sweep Voltammetry" refers to an electrochemical technique that involves sweeping the potential linearly with time while measuring the resulting current response.

The term "Cyclic Voltammetry" refers to an electrochemical technique that involves sweeping the potential back and forth between two set values while measuring the resulting current response.

The term "Potentiometry" refers to an electrochemical technique that involves measuring the potential difference between two electrodes in a solution.

It is noted that the term "bodily fluid sample" may refer to human body fluids such as, but not limited to, urine, tears, saliva, cerebrospinal fluid, bile, semen, vaginal secretions, amniotic fluid, sweat, vitreous humor, blood, serum and/or plasma samples.

## Description

Phosphate levels in blood different than the normal values may indicate the presence of diseases or conditions. High level of phosphate or Pi in blood may be related to chronic renal disease, kidney disease, hypoparathyroidism, increased levels of vitamin D or diabetic ketoacidosis, among others, whereas low levels of phosphates in blood may be related to hyperparathyroidism, malnutrition, alcoholism, or vitamin D deficiency, which in turn is related to osteomalacia.

Therefore, there is a need to measure phosphate levels in blood, serum and/or plasma. In particular, there is a need to find a way to measure phosphate levels which is fast, reliable, simple and inexpensive.

It is known that cobalt electrodes can be used to detect the concentration of phosphate ions in soil or water via potentiometry in a simple and fast way. However, this approach is not fit for detecting phosphate in human blood, serum and/or plasma samples due to the adsorption of proteins on the surface of said electrodes which interferes with the measurements.

As it will be further explained below with the help of the examples, it has been demonstrated a way of overcoming the problem related to the detection of phosphate in blood, serum and/or plasma samples via potentiometry, which is by measuring the current instead of the voltage, using electrodes that comprise cobalt, nickel, tungsten, iron or any of their alloys, as it is shown in the examples.

This is due to the fact that at a pH close to the blood, serum and/or plasma pH, that is, at around 7.4, phosphate ions change the oxidation rate of cobalt and the above-mentioned metals, therefore, the oxidation current will change when the phosphate concentration in the sample changes. It is then possible to obtain, by interpolation and/or extrapolation, a correlation between the oxidation current and the phosphate concentration. In particular, at a pH close to the blood, serum or plasma pH, a higher concentration of phosphate ions decreases the oxidation rate of cobalt.

Also, the adsorption of blood, serum and/or plasma proteins on the surface of the electrodes, which interferes with the potentiometric measurements, may be reduced by diluting and/or changing the pH of the sample, in particular by reducing it, as it is further explained below with the help of experimental data.

In this invention, as is shown in the examples, it is disclosed a method and a device to solve the above-mentioned problems, which is able to detect and to measure phosphate levels in bodily fluids, preferably in blood, serum and/or plasma, more preferably in blood, presenting the following advantages:

- Phosphate detection sensitivity comprising the normal human blood, serum and/or plasma range, that is, for an adult, at least between 0.81 and 1.45 mmol/L.

- Phosphate detection directly in untreated blood, serum and/or plasma samples, without the need to dilute the sample, treat the sample or add reagents.
- Direct reaction between the sample and the electrodes of the phosphate sensor, avoiding the use of enzymes or any other intermediate step.
- Simple electrodes comprising easily available materials, avoiding complex or nanostructured electrodes and materials.

In order to demonstrate these advantages, in the examples shown below, the phosphate concentration is measured on human plasma samples, or artificial serum samples that imitates the properties of human serum. This artificial serum contains bovine serum albumin (BSA) proteins to reproduce the interaction of human serum proteins with the phosphate sensor electrodes.

A way of determining the concentration of phosphate is by obtaining a dependence of phosphate concentration with the redox current measured. The redox current was preferably measured using amperometry or voltammetry techniques. In particular, in example 1, the oxidation current was measured via chronoamperometry. The results of the measurements of example 1 have been plotted in Figure 3, which shows the oxidation current of a cobalt wire produced by successive additions of phosphate ions in artificial serum containing BSA, for different constant voltages applied, i.e. -0.2, -0.3 and -0.4 V respectively. However, it is noted that depending on the voltage and the electrodes, a reduction current may also be measured to determine the phosphate concentration.

As it can be appreciated in Figure 3, there is a clear decreasing trend of the oxidation current intensity as the phosphate concentration increases. On the contrary to the potentiometric approach, the response, in this case, is enough to be used as a method for the determination of phosphate ions in human samples. This trend in the plotted dots for all three voltages is almost linear, which allows for an easy fitting and modelling, which in turn can be used as a calibration curve that correlates the phosphate concentration with a given measured current intensity by using the sensor or method of the invention.

Another way of measuring the phosphate concentration and/or obtaining a calibration curve is by using voltammetry techniques. In particular, in example 2, Linear Sweep Voltammetry was employed to obtain the current values plotted in Figure 4, which shows the measurement of oxidation current as a function of phosphate ions concentration in human plasma using Co-based electrodes. The inset illustrates the good correlation between the concentrations of phosphate ions estimated using a cobalt-based electrode and those using the gold standard method for the determination of phosphate in human samples. All these results suggest that the approach of the invention might be successfully applied to the determination of phosphate ions in other complex matrices such as human blood.

It is possible to successfully determine the phosphate concentration using different configurations for the electrodes, such as electrodeposited, bulk or screen-printed electrodes. In particular, in example 3, it is demonstrated that a calibration curve may also be obtained for screen-printed electrodes. The oxidation current as a function of phosphate concentration, for electrodes made with different inks containing cobalt powder is shown in Figure 5 for samples derived from artificial serum containing BSA. In Figure 5, it can be clearly observed the almost linear dependency of oxidation current versus phosphate concentration, which would allow for obtaining a phosphate concentration or a calibration curve for use in screen-printed phosphate sensors according to the invention.

Not only the device and method of the invention can be carried out with different fabrication processes and different types of electrodes, but also with different materials. For instance, in examples 4, 5 and 6 it is demonstrated the use of other materials such as iron, nickel and tungsten, respectively, to detect and measure the phosphate concentration with the device and method of the invention. Calibration curves obtained with these materials are shown in Figures 6, 7 and 8 respectively. Again, it is observed an almost linear correlation between oxidation current and phosphate concentration, which, in turn, allows for the determination of phosphate concentration for a given oxidation current measured.

In example 7 it is shown that the method and device of the invention provide adequate results to determine the phosphate concentration in three different random human plasma samples, from current measurements as a function of applied voltage. Figure 9 shows the good agreement between the fit (grey line) of the current measurements on three plasma samples (grey circles) and the current measurements of the calibration data (black squares), consisting of four standard plasma samples, which seem to all follow the same linear dependency.

Therefore, the results provided indicate the suitability of the device and method of the invention to measure the concentration of phosphate in human and artificial serum, plasma and/or human blood by measuring the current under an applied potential, using electrodes comprising cobalt, iron, nickel, tungsten or their alloys, and with different electrode configurations such as bulk metal, wire-shaped, electrodeposited and/or screen-printed.

In example 8 it is shown the possibility of determining the phosphate concentration via potentiometry, i.e. by measuring the voltage. Previously, the samples were diluted in an acid solution to reduce the pH below 7.4, as it is further explained in example 8. Figure 10 depicts a comparison of the results obtained by using the potentiometric and colorimetric approach. As it can be seen, the potentiometric determination using cobalt electrodes has an acceptable correlation with the colorimetric assay. This suggests that, by selecting an adequate buffer, is possible to perform the potentiometric

determination of phosphate ions in human blood, serum and/or plasma samples using cobalt electrodes.

The results provided indicate the suitability of the device and method of the invention to measure the concentration of phosphate in human and artificial serum and/or plasma, and/or human blood by measuring the voltage after decreasing the pH of the sample below 7.4, preferably between 1 and 6, more preferably between 3.4 and 4.4, and using electrodes comprising cobalt, iron, nickel, tungsten or their alloys, and with different electrode configurations such as bulk metal, wire-shaped, electrodeposited and/or screen-printed.

Based on the results obtained in the examples, a first aspect of the invention relates to a method for determining the presence and/or the concentration of phosphate in an isolated bodily fluid sample, preferably blood, serum and/or plasma sample, more preferably blood sample. Preferably, the blood, serum and/or plasma is human blood and/or human serum or plasma, or artificial serum or plasma imitating the properties of human serum or plasma. It is understood that, by determining the concentration, it can be determined as well the presence of phosphate in the sample, or at least it can be determined as long as its concentration lies within the detection ranges of the sensor employed to carry out the method of the invention.

In a preferred embodiment of the invention, the method for determining the presence and/or concentration of phosphate in an isolated bodily fluid sample (20), preferably blood, serum and/or plasma, more preferably blood (20), comprises the step of:

a. providing the isolated bodily fluid sample (20), preferably blood, serum and/or plasma sample (20), more preferably blood sample (20) in a sampling area (22), the sampling area (22) comprising at least part of the surface of a first electrode (10), preferably the sampling area (22) also comprising at least part of the surface of a second electrode (12), and the method further comprises either one of the following steps:

b. applying a voltage between the first electrode (10) and a second electrode (12) and measuring a current between said first (10) and second (12) electrodes, or,

c. measuring a voltage between the first electrode (10) and the second electrode (12),

wherein the presence and/or concentration of phosphate in the isolated bodily fluid sample (20), preferably blood, serum and/or plasma sample (20), more preferably blood sample (20), is determined by the current or the voltage measured between the first electrode (10) and the second electrode (12), wherein at least the first electrode (10) is characterized by comprising a metal from the list consisting of cobalt, nickel, tungsten, iron or any of their alloys, preferably cobalt, and at least part of the first electrode (10) is further configured to generate a current associated to a direct redox reaction between said at least part of the first electrode (10) and the provided isolated bodily fluid sample (20), preferably blood, serum and/or plasma sample (20), more preferably blood sample (20), and wherein if step c is performed, prior to said step of measuring the voltage between the first electrode (10) and the second electrode (12), the pH of the isolated bodily fluid sample (20), preferably blood, serum and/or plasma sample (20), more preferably blood sample (20), is reduced below 7.4, preferably between 1 and 7, more preferably between 3.4 and 5.4.

It is noted that if a reference electrode (14) is employed in the method of the invention, the voltage applied in step (b) would be applied between the working electrode (10) and the reference electrode (14), instead of between the working electrode (10) and the counter electrode (12), wherein the current is measured between the first electrode (10) and the second electrode (12).

It is noted that all pH values aforementioned may vary by $\pm$ 30 %, $\pm$ 25 %, $\pm$ 20 %, $\pm$ 15 %, $\pm$ 10 %, $\pm$ 5 % and $\pm$ 1 %.

It is further noted that throughout this document the working electrode (10) is referred to as the "first electrode", and the counter electrode (12) is referred to as the "second electrode". Therefore, in a two-electrode configuration the voltage is applied between the first (10) and the second (12) electrodes, and the current is measured between the first (10) and the second (12) electrodes. However, in a three electrodes configuration, comprising a reference electrode (14) in addition to the first (10) and second (12) electrodes, the voltage would be applied between the first (10) and the reference (14) electrode, and the current would be measured between the first (10) and the second (12) electrodes. It is noted that in this three electrodes configuration, the second electrode (12) may be referred to as counter electrode or as auxiliary electrode. Also, the first electrode (10) may be referred to as working electrode or indicator electrode. Therefore, with these considerations, the method of the invention can be performed both in a two electrodes configuration and/or in a three electrodes configuration.

It is also noted that the pH of a blood, serum and/or plasma sample has generally a value around 7.4, therefore it may require the addition of chemicals to lower said pH, such as acids, preferably the samples are diluted in a potassium hydrogen terephthalate (KHP) buffer. However, the skilled person may know other substances and buffers to lower the pH of the sample, and that are fit for human, serum and/or plasma samples, such as, but not restricted to, HCl, $H_2SO_4$, $HNO_3$, $H_3PO_4$, $CH_3COOH$, citric acid, tartaric acid, lactic acid, malic acid, oxalic acid, etc.

The isolated blood, serum and/or plasma sample (20) may have the pH reduced below physiological value, which lies between 7.35 and 7.45, preferably may have the pH reduced to a value between 1 and 7, more preferably between 3.4 and 5.4, even more preferably to 4.4, before or after providing the isolated blood, serum and/or plasma sample (20) in a sampling area (22), but preferably before measuring a voltage between the first electrode (10) and a second electrode (12). Preferably the sample has the pH reduced before providing said sample (20) in the sampling area (22).

It is also noted that the examples provided refer to electrodes comprising cobalt, nickel, tungsten, iron or their alloys, however, the invention may be extended to electrodes comprising other transition metals that may provide a similar response.

Advantageously, this method provides different ways to measure phosphate levels in a bodily fluid sample (20), preferably blood, serum and/or plasma sample (20), more preferably blood sample (20), that are fast, reliable, simple and inexpensive. In particular, it allows for the determination of the phosphate level in blood, serum and/or plasma by a correlation with either the voltage or the current associated to the reaction. Furthermore, the sample measured by this method may be undiluted and unmodified (when measuring the current), and the method is based in a direct redox reaction that does not need enzymes or intermediate agents. This method employs simple electrodes, with fabrication processes well known to the skilled person, and does not require to fabricate complex electrodes or nanostructures. Also, this method does not require new or complicated materials, as it uses electrodes based on simple, available and easy-to-fabricate materials, such as cobalt, nickel, iron, tungsten or their alloys.

In a first alternative of the first aspect of the invention, the presence and/or concentration of phosphate in an isolated bodily fluid sample (20), preferably blood, serum and/or plasma sample (20), more preferably blood sample (20), is determined by the voltage measured between the first electrode (10) and the second electrode (12), said voltage associated to a direct redox reaction between said at least part of the first electrode (10) and the provided isolated bodily fluid sample (20), preferably blood, serum and/or plasma sample (20), more preferably blood sample (20), wherein the first electrode (10) is, in this case, referred as the indicator electrode (10), and the second electrode is, in this case, referred as the reference electrode (12). It is noted that if a 3-electrode configuration is employed in the method of the invention, the voltage measured would be measured between the first or indicator electrode (10) and the reference electrode (14), instead of between the first or indicator electrode (10) and the counter or second electrode (12).

Therefore, according to a preferred embodiment of the first alternative of the first aspect of the invention, the method for determining the presence and/or concentration of phosphate in an isolated bodily fluid sample (20), preferably blood, serum and/or plasma sample (20), more preferably blood sample (20), comprises the steps of:

a. providing the isolated bodily fluid sample (20), preferably blood, serum and/or plasma sample (20), more preferably blood sample (20), in a sampling area (22), the sampling area (22) comprising at least part of the surface of a first electrode (10),

b. measuring a voltage between the first electrode (10) and a second electrode (12),

wherein the presence and/or concentration of phosphate in the isolated bodily fluid sample (20), preferably blood, serum and/or plasma sample (20), more preferably blood sample (20), is determined by the voltage measured between the first electrode (10) and the second electrode (12), wherein at least the first electrode (10) is characterized by comprising a metal from the list consisting of cobalt, nickel, tungsten, iron or any of their alloys, preferably cobalt, and at least part of the first electrode (10) is further configured to generate a current associated to a direct redox reaction between said at least part of the first electrode (10) and the provided isolated bodily fluid sample (20), preferably blood, serum and/or plasma sample (20), more preferably blood sample (20), and wherein the pH of the isolated bodily fluid sample (20), preferably blood, serum and/or plasma sample (20), more preferably blood sample (20), is reduced below 7.4, preferably between 1 and 7, more preferably between 3.4 and 5.4, prior to measuring the voltage between the first electrode (10) and the second electrode (12).

It is noted that all pH values afore mentioned may vary by a ± 30 %, ± 25 %, ± 20 %, ± 15 %, ± 10 %, ± 5 % and ± 1 %.

It is noted that if a reference electrode (14) is employed in the method of the invention, the voltage measured in step (b) would be measured between the working electrode (10) and the reference electrode (14), instead of between the working electrode (10) and the counter electrode (12).

It is also noted that the pH of a blood, serum and/or plasma sample has generally a value around 7.4, therefore it may require the addition of chemicals to lower said pH, such as acids, preferably the samples are diluted in a KHP buffer. However, the skilled person may know other substances and buffers to lower the pH of the sample, and that are fit for human, serum and/or plasma samples, such as, but not restricted to, HCl, $H_2SO_4$, $HNO_3$, $H_3PO_4$, $CH_3COOH$, citric acid, tartaric acid, lactic acid, malic acid, oxalic acid, etc.

In some embodiments, the isolated blood, serum and/or plasma sample (20) may have the pH reduced below physiologic value, which lies between 7.35 and 7.45, preferably may have the pH reduced to a value between 1 and 7, more

preferably between 3.4 and 5.4, even more preferably to 4.4, before or after providing the isolated blood, serum and/or plasma sample (20) in a sampling area (22), but always before measuring a voltage between the first electrode (10) and a second electrode (12). Preferably the sample has the pH reduced before providing said sample (20) in the sampling area (22).

Preferably, both the first electrode (10), or indicator electrode, and the second electrode (12), or auxiliary electrode, are in contact with the isolated blood, serum and/or plasma sample (20). Wherein the second electrode (12) is preferably configured to maintain a stable and known potential, more preferably by comprising materials that do not react with the phosphate of the sample (20), or by comprising ion-selective membranes to block the flow through said membrane to phosphate ions. In another embodiment of the invention, the membrane allows only specific ions to pass through and interact with the second (12) electrode, while preferably excluding interference from other ions, such as, but not restricted to, phosphate ions.

In order to measure the potential difference between the first (10) and second (12) electrodes, preferably a voltmeter or a potentiometer is connected between the first (10) and second (12) electrodes to measure the potential difference between them. Preferably, the experimental technique employed is potentiometry. The voltmeter or potentiometer provides a readout of the voltage generated by the electrochemical reaction. In other embodiments of the invention, also a control unit is connected to the phosphate sensor (1).

When the isolated bodily fluid sample (20), preferably blood, serum and/or plasma sample (20), more preferably blood sample (20), gets in contact with the first (10), or indicator, electrode, it undergoes an electrochemical reaction at the electrode surface. This reaction involves the transfer of electrons between the sample and the first (10) electrode, resulting in a potential difference between the first (10) and second (12) electrodes. The voltage measured by the potentiometer reaches a stable value once the electrochemical reaction reaches equilibrium. In the case of the first electrode (10) comprising cobalt at pH 4, said electrode oxidizes faster as the concentration of phosphate ions increases, resulting in a decrease of the open circuit potential (OCP) of the system, therefore this voltage is inversely proportional to the concentration of the analyte in the solution or sample (20). However, it is noted that at pH such as 7.4, the cobalt electrode would oxidize slower as the concentration of phosphate ions increase, therefore a different correlation may be stablished. Similarly, for nickel, tungsten, iron or any of their alloys, the relationship of the concentration of phosphate ions with the oxidation of the electrode would depend on the pH.

Before using the phosphate sensor (1), preferably it is calibrated by establishing a relationship between the measured potential and the phosphate concentration. This is carried out by measuring the potential for different known phosphate concentrations and constructing a calibration curve or equation. Once calibrated, the phosphate sensor (1) may be used to measure the potential difference in an unknown blood, serum and/or plasma sample (20). By referring to the calibration curve or equation, the concentration or the presence of the phosphate in the sample (20) can be determined based on the measured potential.

Advantageously, this method provides a quick way to measure phosphate levels in a blood, serum and/or plasma sample that is fast, reliable, simple and inexpensive. In particular, it allows for the determination of the phosphate level in blood, serum and/or plasma by a correlation with the voltage associated with the redox reaction. Furthermore, the method is based on a direct redox reaction that does not need enzymes or intermediate agents. This method employs simple electrodes, with fabrication processes well known to the skilled person, and do not require to fabricate complex electrodes or nanostructures. Also, this method does not require new or complicated materials, as it uses electrodes based on simple, available and easy-to-fabricate materials, such as cobalt, nickel, iron, tungsten or their alloys.

In a second alternative of the first aspect of the invention, the presence and/or concentration of phosphate in an isolated bodily fluid sample (20), preferably blood, serum and/or plasma sample (20), more preferably blood sample (20), is determined by the measured current between the first electrode (10) and the second electrode (12), said current associated to a direct redox reaction between said at least part of the first electrode (10) and the provided isolated bodily fluid sample (20), preferably blood, serum and/or plasma sample (20), more preferably blood sample (20), wherein the first electrode is, in this case, referred as the working electrode (10), and the second electrode is, in this case, referred as the counter electrode (12).

Therefore, according to a preferred embodiment of the second alternative of the first aspect of the invention, the method for determining the presence and/or concentration of phosphate in an isolated bodily fluid sample (20), preferably blood, serum and/or plasma sample (20), more preferably blood sample (20), comprises the steps of:

a. providing the isolated bodily fluid sample (20), preferably blood, serum and/or plasma sample (20), more preferably blood sample (20), in a sampling area (22), the sampling area (22) comprising at least part of the surface of a first electrode (10),

b. applying a voltage between the first electrode (10) and a second electrode (12),

c. subsequently or simultaneously to step b, measuring a current between the first electrode (10) and the second

electrode (12),

wherein the presence and/or concentration of phosphate in the isolated bodily fluid sample (20), preferably blood, serum and/or plasma sample (20), more preferably blood sample (20), is determined by the measured current between the first electrode (10) and the second electrode (12), and wherein at least the first electrode (10) is characterized by comprising a metal from the list consisting of cobalt, nickel, tungsten, iron or any of their alloys, preferably cobalt, and at least part of the first electrode (10) is further configured to generate a current associated to a direct redox reaction between said at least part of the first electrode (10) and the provided isolated bodily fluid sample (20), preferably blood, serum and/or plasma sample (20), more preferably blood sample (20).

It is noted that if a reference electrode (14) is employed in the method of the invention, the voltage applied in step (b) would be applied between the working electrode (10) and the reference electrode (14), instead of between the working electrode (10) and the counter electrode (12).

It is noted that by sampling area we refer to a surface or volume comprising said surface where the sample can be deposited, in such a way that the electrodes are in contact with the sample. Preferably, at least the first electrode, herein after referred as the working electrode (10), is in contact with the sample. More preferably, at least the working electrode (10) and the second electrode, herein after referred as the counter electrode (12), are in contact with the sample. Alternatively, a reference electrode (14) may be employed, in which case the voltage is applied between the first or working electrode (10) and the reference electrode (14), and the current is measured between the first or working electrode (10) and the second or counter electrode (12). However, a skilled person may find different electrode configurations that can allow the method to be carried out, as long as the circuit is complete, and the charge can flow during the redox reaction. For instance, other auxiliary electrodes may also be in contact with the sample.

It is also noted that there are different ways to apply a voltage between the working electrode (10) and the counter electrode (12), which generally comprise using a voltage source or power supply, a potentiostat or a galvanostat, appropriate metallic connections to said electrodes and the corresponding cables and wiring to connect all elements that need electrical communication. Preferably, it also includes a control unit or electronic means to control the different parameters, such as voltage or time, among others. The specific devices and connections may vary depending on the electrochemical phosphate sensor (1) configuration used and the measurement technique being employed.

It is further noted that there are different ways to measure a current between the working electrode (10) and the counter electrode (12), which generally comprise using an ammeter, also known as current meter or current amplifier, or a multimeter, as well as a data acquisition system comprising hardware and software used to acquire and record data from various sensors, including current measurements, and the corresponding cables and wiring to connect all elements that need electrical communication. The specific devices and connections may vary depending on the electrochemical phosphate sensor (1) configuration used and the measurement technique being employed.

As was explained before, the presence of phosphate in the blood, serum and/or plasma sample (20) changes the oxidation rate of the working electrode (10). On the other hand, when a voltage is applied between the working (10) and counter (12) electrodes, it creates an electric field that drives the movement of charged species in the electrolyte solution (20), therefore, the applied voltage also affects the rate of the redox reaction. At the working electrode (10), the redox reaction involves the transfer of electrons between the electrode (10) and the species in the solution (20), and this electron transfer, in turn, generates an electrical current that can be measured, and which will show a different value depending on the phosphate concentration and the voltage applied.

By sweeping the potential across a range, as done in cyclic voltammetry, it allows for the determination of the current response as a function of voltage, and may allow the determination of the potential at which the current associated to a redox reaction is maximum. The resulting current response, as well as its maximum and minimum values, provides information about the concentration of the analyte, the kinetics of the redox reaction, and the reversibility of the process. Also, by keeping the voltage constant, as done in amperometry, it allows for the determination of an oxidation or a reduction current value for a given voltage, wherein the oxidizing or reducing character of the current will depend on the value of the voltage applied. Varying the phosphate concentration then allows to elaborate a calibration curve that can be used to determine the presence and/or concentration of phosphate in the sample.

It is noted that this current response has been demonstrated in the examples for working electrodes (10) that comprise cobalt, nickel, tungsten, iron or any of their alloys, however, it is possible that the skilled person could find other materials with a similar electrochemical response that would allow to measure the current between the working (10) and the counter (12) electrodes under an applied potential, for instance, other transition metals. Regarding the counter electrode (12), it may comprise one or more different materials, preferably inert materials, such as platinum, gold, glassy carbon, Carbon-based materials, conductive metal oxides or conductive polymers. Also, the counter electrode (12) preferably has a bigger size and bigger surface than the working electrode (10) in order to avoid limiting the reaction at the counter electrode (12), however, in some configurations the counter electrode (12) may have a similar or smaller size and/or surface, as long as it does not limit the reaction.

The reaction above mentioned between the sample (20) and the working electrode (10) is preferably a direct

redox reaction that occurs directly at the electrode-electrolyte interface, without the involvement of any additional species or mediator molecules, such as ferrocene derivatives, and it involves the transfer of electrons between the electrode (10) and the electrolyte species comprised in the sample (20).

It is further noted that throughout this document, when referring to the sample (20), it is often referred to as a blood, serum and/or plasma sample (20), however it is understood that the method and the device of the invention may be applied to any other bodily fluid sample (20), since other bodily fluid samples are simpler and contain less proteins and/or species that could interfere with the measurements than blood samples.

Advantageously, this method provides a way to measure phosphate levels in bodily fluids, preferably blood, serum and/or plasma that is fast, reliable, simple and inexpensive. Furthermore, the sample measured by this method may be undiluted and unmodified, and the method is based in a direct redox reaction that does not need enzymes or intermediate agents. This method employs simple electrodes, with fabrication processes well known to the skilled person, and do not require to fabricate complex electrodes or nanostructures. Also, this method does not require new or complicated materials, as it uses electrodes based on simple, available and easy to fabricate materials, such as cobalt, nickel, iron, tungsten or their alloys.

In a preferred embodiment of the method of the first aspect of the invention, the method further involves using a reference electrode (14). In this case the voltage of step (b) is applied between the first electrode (10) and the reference electrode (14), instead of between the first (10) and second (12) electrodes. However, the current is still measured between the first (10) and the second (12) electrodes. Regarding the voltage measured in step (c), said voltage would be measured between the first electrode (10) and the reference electrode (14), instead of between the first (10) and the second (12) electrodes.

It is noted that preferably the reference electrode (14) maintains a constant potential against which the working electrode's (10) potential is measured, enhancing the precision of the voltage or current measurement. Alternatively, the skilled person may adapt this method to other electrode configurations, such as a single electrode system or a four-electrode system. Moreover, the concept is not limited to existing configurations but extends to any electrode arrangements that may be developed in the future. Materials for electrodes may include, but are not limited to, metals such as cobalt, nickel, tungsten, iron, or their alloys, with cobalt being preferred.

Advantageously, employing a three-electrode configuration (comprising working electrode (10), counter or auxiliary electrode (12), and reference electrode (14)) offers several advantages for sensitive and precise electrochemical measurements over a two-electrode configuration (comprising a working electrode (10) and a counter electrode (12)), such as increased precision and reproducibility of the results. According to a preferred embodiment of the invention, at least part of the surface of the working electrode (10) comprises a permselective membrane that allows the flow through said membrane of at least phosphate ions, wherein said permselective membrane preferably blocks the flow through said membrane of at least a reducing agent, and oxidizing agent, inorganic and/or organic species, a protein and/or a biomolecule. In some embodiments of the invention, a permselective membrane may surround any of the electrodes, preferably the working electrode (10), and this membrane may be the same or different for each electrode. In some other embodiments of the invention the permselective membrane may surround the sampling area (22) or volume comprising said sampling area (22), or adopt any configuration that allows the sample to pass through said membrane before getting in contact with any part of the working electrode (10). Said permselective membrane may comprise any material adequate to carry out the task of selectively allow the flow through said membrane of the electrolytes in the sample, such as, but not limited to, Nafion, poly(vinyl alcohol), cellulose acetate, chitosan, ion-exchange membranes based on sulfonated polymers or polyelectrolyte multilayers, or molecularly imprinted polymers. In some other embodiments of the invention, the permselective membrane blocks the flow through said membrane of at least a reducing agent and/or biomolecule, or the combination of at least a reducing and at least an oxidizing agent and/or biomolecule. According to another preferred embodiment of the invention, the permselective membrane may be configured to block the flow through said membrane to phosphate ions, preferably while allowing the flow through the membrane of at least another reducing agent, oxidizing agent, inorganic species, protein and/or biomolecule, more preferably allowing the flow through the membrane of any another reducing agent, oxidizing agent, inorganic species, protein and/or biomolecule different than phosphate. In another preferred embodiment of the invention, said permselective membrane may be controlling the flow of the sample only towards a reference electrode (14) comprised in the phosphate sensor (1) of the invention, that is; it may be comprised, surrounding, protecting or covering only the reference electrode (14), to filter out the phosphate in the part of the sample (20) that may reach the reference electrode (14), in order to obtain a reference measurement of the blood, serum and/or plasma sample (20).

There are several advantages from using a membrane, from improving the quality and the precision of the measurements, to increase the reproducibility and therefore the reliability of the sensor (1). In particular, by introducing the permselective membrane in the working electrode (10), it is possible to reduce the presence of interfering species such as biomolecules, proteins, inorganic molecules or oxidizing or reducing agents, and, therefore, reducing the noise, increasing the signal, and improving reproducibility. By introducing the permselective membrane in other electrodes, such as the reference electrode (14), it is possible to obtain a more stable potential to use as reference, since interfering species such

as biomolecules, proteins, inorganic molecules or oxidizing or reducing agents can be filtered. Also, it is possible to use the permselective membrane in the reference electrode (14) to only block phosphate ions, in order to provide a reference value of a redox current or redox potential which is not hindered by the presence of phosphate, and therefore having a reference of the reaction rate in the sample (20) without the presence of phosphate.

Advantageously, the presence of the permselective membrane allows to filter out agents and/or biomolecules that may interfere with the reaction at the interface electrode/electrolyte, whereas it allows the flow of the phosphate of interest. This way, it is possible to obtain more reliable, precise and robust measurements, such that different levels of different oxidising or reducing agents may not interfere with the measurements.

In a preferred embodiment of the method of the invention, the determination of the presence and/or concentration of phosphate in blood, serum and/or plasma is performed by comparing the value of the current measured with reference values of current versus phosphate concentration, preferably the reference values are obtained from a dependency between the current and the concentration of phosphate, more preferably this dependency is linear or polynomial. It is noted that said reference values may comprise a calibration curve, a set of points, an interpolation or an extrapolation of a curve or a set of points, or a formula, which allows the skilled person to obtain the phosphate concentration by measuring the current intensity and comparing with the calibration data or curve. Said calibration data or curve may be linear, polynomic, or present any other mathematical expression that is a good fit to the reference values or the data points of the calibration curve. The current measured may be the redox current associated with a direct redox reaction between the working electrode (10) and the provided blood, serum and/or plasma sample (20). In some embodiments of the invention, the current measured associated with a direct redox reaction between the working electrode (10) and the provided blood, serum and/or plasma sample (20) is hindered by the presence of phosphate in the sample (20), therefore, there is an inverse relationship between the rate of the redox reaction taking place and the concentration of phosphate in the sample (20).

Advantageously, obtaining this dependency between the phosphate concentration and the current measured due to the redox process taking place at the interface electrode/electrolyte allows to obtain the phosphate concentration from a measurement of the redox current, by comparing the reference values with the measured values.

According to another preferred embodiment of the method of the invention, the sampling area (22) further comprises at least part of the surface of a reference electrode (14). It is noted that this reference electrode (14) may be protected, encapsulated, enclosed or separated from the sample by an impervious layer or by any other means, such as a permselective membrane, preferably a permselective membrane that blocks the flow through said membrane to phosphate ions. It is also noted that the reference electrode (14) may be in contact with a reference solution, different from the sample and also isolated from this one. Therefore, the phosphate sensor (1) used in the method of the invention may comprise a reference electrode (14). Preferably the phosphate sensor (1) used in the method of the invention comprises a reference electrode in addition to the working electrode (10) and the counter electrode (12). Additionally, the phosphate sensor (1) of the invention may also comprise one or more auxiliary electrodes.

Advantageously, comprising a reference electrode (14) may play a crucial role in an electrochemical sensor by providing a stable reference potential against which the potential of the working electrode (10) is measured. It establishes a fixed reference point for accurate and meaningful electrochemical measurements.

In a preferred embodiment of the method of the invention, the applied voltage is kept constant, preferably at a voltage between -2.0 and +2.0 volts, more preferably between -1.2 and +1.2 volts, even more preferably between -0.6 and +0.4 volts. Preferably, the voltage is kept constant for an adequate amount of time to perform a current measurement, such as, but not limited to, any time value comprised in the range between 0 and 10 seconds, such as 10 s, 5 s, 2 s, 1s, 0.5 s, 0.1 or 0.05 s. It is noted that any quantity for time or voltage aforementioned may vary by a $\pm$ 30 %, by a $\pm$ 20 %, by a $\pm$ 15 %, by a $\pm$ 10 % or by a $\pm$ 5 %. Preferably the voltage is equal or has a bigger absolute value than the open circuit potential (OCP) of the electrochemical sensor. For example, if the OCP is +0.2 V, then preferably the constant voltage applied may be +0.2 V or more positive values, such as +0.5 V, or, if the OCP is -0.3 V, then preferably the constant voltage applied may be -0.3 V or more negative voltages, such as -0.4 V. Some examples of a measurement technique wherein the current is measured while a constant voltage is applied between the working electrode (10) and the counter electrode (12) are amperometry and chronoamperometry.

Advantageously, keeping the voltage constant for an adequate amount of time allows to obtain a measurement of the current that can be related to the amount of phosphate in the sample.

In another preferred embodiment of the method of the invention, the applied voltage is varied with time, preferably it is varied between voltages comprised in the range between -2.0 and +2.0 volts, more preferably between -1.2 and +1.2 volts, even more preferably in the range between -0.6 and +0.4 volts. Preferably, the voltage is varied at a fixed and adequate scan rate to perform a current measurement. Preferably the voltage is varied at a scan rate comprised between 0 and 5 volts per second, more preferably between 0 and 1 volt per second, even more preferably between 0 and 0.5 volts per second, even more preferably between 0 and 0.1 volts per seconds, even more preferably between 0.001 and 0.05 volts per second. It is noted that any quantity for scan rate or voltage aforementioned may vary by a $\pm$ 30 %, by a $\pm$ 20 %, by a $\pm$ 15 %, by a $\pm$ 10 % or by a $\pm$ 5 %. Some examples of a measurement technique wherein the current is measured while a

varying voltage is applied between the working electrode (10) and the counter electrode (12) are voltammetry, linear sweep voltammetry or cyclic voltammetry. It is noted that, in order to relate the measured current as a function of the applied varying voltage, the current corresponding to a particular voltage value is selected and compared with the reference data. Preferably, this voltage is the anodic or the cathodic peak potential, however, this voltage may be any other chosen voltage inside the voltage range.

Advantageously, varying the voltage at an adequate scan rate allows to obtain a measurement of the current that can be related to the amount of phosphate in the sample.

According to another preferred embodiment of the method of the invention, the measured current between the working electrode (10) and the counter electrode (12) under applied voltage is measured via amperometry, preferably chronoamperometry or pulse amperometry, more preferably chronoamperometry, or via voltammetry, preferably Linear-Sweep Voltammetry, Cyclic voltammetry, Normal Pulse Voltammetry, Square-Wave Voltammetry or Differential Pulse Voltammetry, more preferably via Linear-Sweep Voltammetry or Cyclic voltammetry. It is noted that the skilled person may know different techniques for measuring the current as a function of the applied voltage, that may be used for the method of the invention as long as there is a correlation between the measured current value and the phosphate concentration in the sample. Also, it is noted that these experimental techniques may be used alone or in combination to determine the phosphate concentration in a sample, preferably a blood, serum and/or plasma sample, and they also may be used alone or in combination to generate the one or more calibration curves correlating redox current versus voltage applied (varying voltage, or fixed voltage) for different concentrations of phosphate.

Advantageously, using any of these experimental techniques alone or in combination allows for the creation of calibration curves of current intensity versus voltage for different phosphate concentrations, that in turn can be used in the method of the invention to determine the phosphate concentration in a sample, preferably a blood, serum and/or plasma sample.

In a preferred embodiment of the method of the invention, at least the working electrode (10) comprises cobalt in its composition. It is noted that if the phosphate sensor comprises a reference electrode (14), this reference electrode (14) may comprise cobalt in its composition as well. Any other auxiliary electrode or the counter electrode (12) may also comprise cobalt in its composition. Cobalt is a particularly adequate material for the phosphate electrochemical sensor due to the phosphate assisted redox reaction that happens on its surface, and that may be represented in the following way:

$$(1) \qquad \tfrac{1}{2}\, O_2 + 2H^+ + 2e^- \rightleftharpoons H_2O$$

$$(2) \qquad Co + H_2O \rightleftharpoons Co(OH)^+ + H^+ + 2e^-$$

$$(3) \qquad 4Co(OH)^+ + 2H_2PO_4^- \rightleftharpoons Co_3(PO_4)_2 + CoO + 3H_2O + 2H^+$$

$$(4) \qquad 4Co(OH)+ + 2HPO_4^{2}- \rightleftharpoons Co_3(PO_4)_2 + CoO + 3H_2O$$

Advantageously, cobalt has proven to be a very effective and reliable electrode material to measure phosphate concentration in a blood, serum and/or plasma sample (see figures 3 to 6), due to the redox reaction happening in the surface of the electrode comprising cobalt, preferably at the surface of the working electrode (10) and/or the reference electrode (14).

In a preferred embodiment of the method of the invention, the determined concentration of phosphate comprises values within the possible values of phosphate concentration in serum, plasma and/or blood of humans, preferably the concentration of phosphate comprises values between 0.01 to 25 mmol/L, more preferably between 0.2 to 10 mmol/L, even more preferably between 0.3 to 3 mmol/L. Therefore, the phosphate sensor employed in the method of the invention may have a sensibility range adequate to detect and determine the phosphate concentration in human blood, serum or plasma, whether this phosphate concentration is within the range of normal phosphate concentration values in human blood, serum and/or plasma (i.e. between 0.8 and 1.5 mmol/L), or it corresponds to possible, but abnormally high or abnormally low phosphate concentration in human blood, serum or plasma. It is noted that any phosphate concentration aforementioned may vary by a ± 30 %, by a ± 20 %, by a ± 15 %, by a ± 10 % or by a ± 5 %.

Advantageously, having a phosphate sensor with sensitivity within the range of possible phosphate concentration values in human blood, serum and/or plasma allows to determine if said phosphate concentration lies within the normal values of phosphate concentration in human serum, plasma and/or blood (i.e. between 0.8 and 1.5 mmol/L) or if it is abnormally high or abnormally low, which can provide useful information regarding the health status of a patient.

In a preferred embodiment of the method of the invention, at least the working electrode (10) is screen printed, electrodeposited or comprises a piece of bulk metal, wherein preferably said piece of bulk metal has the shape of a wire, a sheet, a plate, a disc or any other adequate bulk metal shape. It is noted that any other electrode, such as the counter electrode (12), the reference electrode (14) or any other auxiliary electrode may also be screen printed, electrodeposited or comprises a piece of bulk metal, wherein preferably said piece of bulk metal has the shape of a wire, a sheet, a plate, a disc or any other adequate bulk metal shape. It is noted as well that the skilled person may know many different ways of fabricating electrodes, that can be applied to the phosphate sensor of the method of the invention, such as, but not limited to, interdigitated electrodes, microelectrodes, nanoelectrodes, etc

Advantageously, the above-mentioned shapes for the electrodes used in the method of the invention are adequate to react with the blood, serum and/or plasma sample and to obtain a redox current intensity for a given voltage that, when compared with a reference curve or reference values, allows to determine the phosphate levels in the sample.

According to another preferred embodiment, a method for determining the presence and/or concentration of phosphate *in vivo* in blood, serum and/or plasma of a patient comprises the steps of:

a. providing at least part of the surface of a working electrode (10) in contact with live tissue of the patient intradermally, wherein said part of the surface of a working electrode (10) is configured to get in contact with blood of the patient,

b. applying a voltage between the working electrode (10) and a counter electrode (12);

c. subsequently or simultaneously to step b, measuring a current between the working electrode (10) and the counter electrode (12),

wherein the presence and/or concentration of phosphate in the isolated blood, serum and/or plasma sample is determined by a measured current between the working electrode (10) and the counter electrode (12), and wherein at least the working electrode (10) is characterized by comprising a metal from the list consisting of cobalt, nickel, tungsten, iron or any of their alloys, and at least part of the working electrode (10) is further configured to generate a current associated to a direct redox reaction between said at least part of the working electrode (10) and the provided isolated blood, serum and/or plasma sample (20).

It is noted that at least part of the working electrode (10) may be comprised in a lab on a chip, a patch, or any other sensor and/or biosensor with means of at least partial intradermal placement.

Advantageously, this way the patient can have the phosphate levels monitored in real time.

According to a second aspect of the invention, a phosphate sensor is disclosed. In one embodiment of the invention, it is disclosed a phosphate sensor (1) for determining the presence and/or concentration of phosphate in a blood, serum and/or plasma sample (20). The phosphate sensor (1) comprising a first (10) electrode and preferably a second (12) electrode, more preferably further comprising a reference electrode (14), and wherein the phosphate sensor further comprises and a sampling area (22), said sampling area (22) comprising at least part of the surface of a first electrode (10), wherein said first electrode (10) is configured to be at least partially in contact with the blood, serum and/or plasma sample (20) provided in the sampling area (22), wherein said first electrode (10) is further configured to generate a current associated to a direct redox reaction between said at least part of the first electrode (10) and the provided isolated blood, serum and/or plasma sample (20), and wherein at least the first electrode (10) comprise a metal from the list consisting of cobalt, nickel, tungsten, iron or their alloys, preferably cobalt.

It is noted that the first electrode (10) may be configured to have one of its surfaces completely in contact with the blood, serum and/or plasma sample (20), in order to improve reproducibility of the measurements and decrease the variability of the open circuit potential (OCP) in the phosphate sensor (1).

It is also noted that preferably a voltage is applied between the first electrode (10) and the second electrode (12), generating a redox current between the first electrode (10) and the second electrode (12). In some embodiments of the sensor (1) of the invention, a reference electrode (14) is comprised, in which case, a voltage is applied between the first electrode (10) and the reference electrode (14) instead of between the first (10) and second (12) electrodes, which generates a redox current between the first (10) electrode and the second (12) electrode.

According to a first alternative of the second aspect to the invention, the phosphate sensor further comprises a reference electrode (14), and the presence and/or concentration of phosphate in the isolated blood, serum and/or plasma sample (20) is determined by a voltage measured between the first electrode (10) and the reference electrode (14)said voltage associated to a direct redox reaction between said at least part of the first electrode (10) and the provided isolated blood, serum and/or plasma sample (20).. Preferably, the pH of the isolated blood, serum and/or plasma sample (20) is reduced below 7.4, more preferably between 1 and 7, even more preferably between 3.4 and 5.4, prior to measuring the voltage between the first electrode (10) and the second electrode (12).

It is noted that all pH values afore-mentioned may vary by a ± 30 %, ± 25 %, ± 20 %, ± 15 %, ± 10 %, ± 5 % and ± 1 %.

It is also noted that the pH of a blood, serum and/or plasma sample has generally physiological values, i.e. 7.4 or between 7.35 and 7.45, therefore it may require the addition of chemicals to lower said pH, such as acids, preferably the samples are diluted in a KHP buffer. However, the skilled person may know other substances and buffers to lower the pH of the sample, and that are fit for human blood, serum and/or plasma samples, such as, but not restricted to, HCl, $H_2SO_4$, $HNO_3$, $H_3PO_4$, $CH_3COOH$, citric acid, tartaric acid, lactic acid, malic acid, oxalic acid, etc.

The isolated blood, serum and/or plasma sample (20) may have the pH reduced below 7.4, preferably between 1 and 7, more preferably between 3.4 and 5.4, even more preferably at 4.4, before or after providing the isolated blood, serum and/or plasma sample (20) in a sampling area (22), but always before measuring a voltage between the first electrode (10) and a second electrode (12). Preferably the sample has the pH reduced before providing said sample (20) in the sampling area (22).

It is noted that the first electrode (10) may be configured to have one of its surfaces completely in contact with the blood, serum and/or plasma sample (20), in order to improve the reproducibility of the measurements and decrease the variability of the open circuit potential in the phosphate sensor (1).

Preferably, both the first electrode (10), or indicator electrode, and the second electrode (12), or counter electrode are in contact with the isolated blood, serum and/or plasma sample (20),and the reference electrode is preferably configured to maintain a stable and known potential, more preferably by comprising materials that do not react with the phosphate of the sample (20), or by comprising ion-selective membranes to block the flow through said membrane to phosphate ions. In another embodiment of the invention, the membrane allows only specific ions to pass through and interact with the second (12) (or reference) electrode, while excluding interference from other ions, such as, but not restricted to, phosphate ions.

In order to measure the potential difference between the first (10) and second (12) electrodes, or between the first (10) and reference (14) electrodes, preferably a voltmeter or a potentiometer is connected between said electrodes to measure the potential difference between them. The voltmeter or potentiometer provides a readout of the voltage generated by the electrochemical reaction. In other embodiments of the invention, also a control unit is connected to the phosphate sensor (1).

When the isolated blood, serum and/or plasma sample gets in contact with the first (10) (or indicator) electrode, it undergoes an electrochemical reaction at the electrode surface. This reaction involves the transfer of electrons between the sample and the first (10) electrode, resulting in a potential difference between the first (10) and second (12) electrodes, in a two-electrode configuration, or between the first (10) and the reference (14) electrodes, in a three-electrodes configuration. The voltage measured by the potentiometer reaches a stable value once the electrochemical reaction reaches equilibrium. In this case, the first electrode (10), comprising cobalt, nickel, tungsten, iron or any of their alloys, preferably cobalt, oxidizes faster as the concentration of phosphate ions increases, resulting in a decrease of the open circuit potential (OCP) of the system, therefore this voltage is inversely proportional to the concentration of the analyte in the solution or sample (20).

Before using the phosphate sensor (1), preferably it is calibrated by establishing a relationship between the measured potential and the phosphate concentration. This is done by measuring the potential for different known phosphate concentrations and constructing a calibration curve or equation. Once calibrated, the phosphate sensor (1) may be used to measure the potential difference in an unknown blood, serum and/or plasma sample (20). By referring to the calibration curve or equation, the concentration or the presence of the phosphate in the sample (20) can be determined based on the measured potential.

Advantageously, this phosphate sensor (1) provides quickly measures phosphate levels in a blood, serum and/or plasma sample in a way that is fast, reliable, simple and inexpensive. In particular, it allows for the determination of the phosphate level in blood, serum and/or plasma by a correlation with the voltage associated with the redox reaction. Furthermore, the method is based in a direct redox reaction that does not need enzymes or intermediate agents. This sensor (1) employs simple electrodes, with fabrication processes well known to the skilled person, and does not require to fabricate complex electrodes or nanostructures. Also, this phosphate sensor (1) does not require new or complicated materials, as it uses electrodes based on simple, available and easy to fabricate materials, such as cobalt, nickel, iron, tungsten or their alloys.

In a second alternative of the second aspect of the invention, the presence and/or concentration of phosphate in the isolated blood, serum and/or plasma sample is determined by the measured current between the first electrode (10) and the second electrode (12), said current associated to a direct redox reaction between said at least part of the first electrode (10) and the provided isolated blood, serum and/or plasma sample (20), wherein the first electrode (10) is, in this case, referred as the working electrode (10), and the second electrode (12) is, in this case, referred as the counter electrode (12).

Therefore, according to a preferred embodiment of the second alternative of the second aspect of the invention, it is disclosed a phosphate sensor (1) for determining the presence and/or concentration of phosphate in a blood, serum and/or plasma sample (20), the phosphate sensor (1) comprising a sampling area (22), said sampling area (22) comprising at least part of the surface of a first electrode (10), herein after known as the working electrode (10), wherein said working

electrode (10) is configured to be at least partially in contact with the blood, serum and/or plasma sample (20) provided in the sampling area (22), wherein the phosphate sensor (1) further comprises a control unit configured to apply a voltage between the working electrode (10) and a second electrode (12), herein after known as the counter electrode (12), wherein said working electrode (10) is further configured to generate a current associated to a direct redox reaction between said at least part of the working electrode (10) and the provided isolated blood, serum and/or plasma sample (20), and wherein at least the working electrode (10) comprise a metal from the list consisting of cobalt, nickel, tungsten, iron or their alloys.

It is noted that the first electrode (10) may be configured to have one of its surfaces completely in contact with the blood, serum and/or plasma sample (20), in order to improve the reproducibility of the measurements and decrease the variability of the open circuit potential in the phosphate sensor (1).

It is noted that, by sampling area, we refer to a surface or volume where the sample can be deposited, in such a way that the electrodes are in contact with the sample. Preferably, at least the working electrode (10) is in contact with the sample. More preferably, at least the working electrode (10) and the counter electrode (12) are in contact with the sample. However, a skilled person may find different electrode configurations that can allow the method to be carried out, as long as the circuit is complete and the charge can flow during the redox reaction. For instance, auxiliary electrodes may also be in contact with the sample.

It is also noted that there are different ways to apply a voltage between the working electrode (10) and the counter electrode (12), which generally comprise using a voltage source or power supply, a potentiostat or a galvanostat, appropriate metallic connections to said electrodes and the corresponding cables and wiring to connect all elements that need electrical communication. Preferably, it also includes a control unit or electronic means to control the different parameters, such as voltage and time, among others. The specific devices and connections may vary depending on the electrochemical phosphate sensor (1) configuration used and the measurement technique being employed.

It is further noted that there are different ways to measure a current between the working electrode (10) and the counter electrode (12), which generally comprise using an ammeter, also known as current meter or current amplifier, or a multimeter, as well as a data acquisition system comprising hardware and software used to acquire and record data from various sensors, including current measurements, and the corresponding cables and wiring to connect all elements that need electrical communication. The specific devices and connections may vary depending on the electrochemical phosphate sensor (1) configuration used and the measurement technique being employed.

As explained before, the presence of phosphate in the blood, serum and/or plasma sample (20) changes the rate of the oxidation reaction at the working electrode (10). On the other hand, when a voltage is applied between the working and counter electrodes, it creates an electric field that drives the movement of charged species in the electrolyte solution, therefore, the applied voltage also affects the rate of the redox reaction. At the working electrode, the redox reaction involves the transfer of electrons between the electrode and the species in the solution, and this electron transfer, in turn, generates an electrical current that can be measured, and which will show a different value depending on the phosphate concentration and the voltage applied.

By sweeping the potential across a range, as done in Cyclic Voltammetry, it allows for the determination of the potential at which the oxidation or reduction occurs. The resulting current response, as well as its maximum and minimum values, provides information about the concentration of the analyte, the kinetics of the redox reaction, and the reversibility of the process. Also, by keeping the voltage constant, as it is in amperometry, it allows for the determination of a redox current value for a given voltage. Varying the phosphate concentration then allows to elaborate a calibration curve that can be used to determine the presence and/or concentration of phosphate in the sample.

It is noted that this current response has been demonstrated in the examples for working electrodes that comprise cobalt, nickel, tungsten, iron or any of their alloys, however, it is possible that the skilled person could find other materials with a similar electrochemical response that would allow to measure the current between the working (10) and the counter (12) electrodes under an applied potential. Regarding the counter electrode (12), it may comprise one or more different materials, preferably inert materials, such as platinum, gold, glassy carbon, carbon-based materials, conductive metal oxides or conductive polymers, among others. Also, the counter electrode (12) usually has a bigger size and bigger surface than the working electrode (10) in order to avoid limiting the reaction at the counter electrode (12), however, in some configurations the counter electrode (12) may have a similar or smaller size and/or surface, as long as they do not limit the reaction.

The reaction above mentioned between the sample (20) and the working electrode (10) is preferably a direct redox reaction that occurs directly at the electrode-electrolyte interface, without the involvement of any additional species or mediator molecules, such as enzymes, and it involves the transfer of electrons between the electrode and the electrolyte species comprised in the sample.

Figure 1 shows a lateral view of a schematic representation of a phosphate sensor (1) according to one or more embodiments of the invention. In Figure 1 we can appreciate that there are three electrodes, namely a working electrode (10), a counter electrode (12) and a reference electrode (14), however in some embodiments of the invention it may be possible to remove the reference electrode (14) and work only with a working electrode (10) and a counter electrode (12). It is noted that the reference electrode (14) shown in Figure 1 is encapsulated, to avoid contact with the sample, however in

other embodiments this encapsulation may be different or may not isolate it from the sample, and may comprise a solution with reference electrolytes, or may comprise a permselective membrane, preferably a permselective membrane that blocks the flow through said membrane at least to phosphate, or a permselective membrane that allows the flow through said membrane at least to phosphate. In some other embodiments of the invention the phosphate sensor may present more electrodes than the ones shown in Figure 1, for example it could comprise also one or more additional auxiliary electrodes, preferably to compensate for any resistance or impedance changes in the electrochemical system. The electrodes shown in Figure 1 have a cylindrical shape, but in some embodiments of the invention they could present other shapes adequate for an electrochemical sensor, for instance they could be a plate, a sheet, a prism or a disc, among many others. Also, in some embodiments of the invention the electrodes may comprise a bulk piece of metal, and adopt any shape adequate for the electrochemical sensor. For instance, in different embodiments of the invention the shape may present different surface/volume ratios to either enhance or decrease the interface interactions between the electrolytes in the sample and the electrodes, for example the surface may be corrugated, present a geometrical shape or a fractal design. The electrodes, as indicated above, may comprise a bulk piece of metal, or they may be fabricated by other techniques such as electrodeposition, screen-printing, or any other technique known to the skilled person. Therefore, the size, aspect ratios and shape of the electrodes and other parts of the electrochemical sensor (1) depicted in Figure 1 have only illustrative purposes, and may vary from one embodiment to another, therefore they may be bigger or smaller, longer or shorter, or thicker or thinner than what is represented in Figure 1, as long as they present an adequate shape.

The sampling area (22) shown in Figure 1 comprises part of the working electrode (10) and part of the counter electrode (12), and is configured to receive the sample and keep it in contact with said electrodes. The reference electrode (14) in Figure 1 is protected by a capsule to avoid contact with the sample, however in other embodiments of the invention it may be protected differently or be placed elsewhere. In some other embodiments of the invention the sampling area may comprise only the working electrode (12), the working (10) and counter (12) electrodes, the working (10) and counter (12) and reference electrode (14), the working (10) and counter (12) and other auxiliary one or more electrodes, or the working (10), counter (12), auxiliary and reference (14) electrodes, as long as they are configured to carry out their respective tasks and the electric circuit is closed, allowing the current to flow. It is noted that this sampling area may adopt different shapes and present different sizes, as long as it maintains the sample in contact with at least the working electrode (10), preferably with at least the working (10) and counter (12) electrodes, more preferably with at least the working (10), counter (12) and reference (14) electrodes.

Figure 2 shows a perspective view of a schematic representation of a screen-printed phosphate sensor (1) according to one or more embodiments of the invention. In figure 2 we can appreciate that there are three electrodes, namely a working electrode (10), a counter electrode (12) and a reference electrode (14), however in some embodiments of the invention it may be possible to remove the reference electrode (14) and keep only a working electrode (10) and a counter electrode (12). It is noted that the reference electrode (14) shown in Figure 2 is protected by a layer to avoid contact with the sample, however in other embodiments this layer of protection may be a permselective membrane, preferably configured to block the flow through said membrane of at least the phosphate, or it may be different, and may include a solution or an aggregate with reference electrolytes, and may comprise materials resistant to corrosion and/or oxidation. In some other embodiments of the invention the phosphate sensor may present more electrodes than the ones shown in Figure 2, for example it could comprise also one or more auxiliary electrodes, preferably to compensate for any resistance or impedance changes in the electrochemical system. The electrodes shown in Figure 2 are screen printed in circular or rectangular shape, but in some embodiments of the invention they could present other shapes adequate for an electrochemical sensor, for instance they could be screen-printed in any geometrical, regular or irregular shape, as long as they present an adequate surface for the electrochemical sensor. For instance, in different embodiments of the invention the screen-printed electrodes may present different surface/volume ratios to enhance or decrease the interface interactions between the electrolytes in the sample and the electrodes, for example the surface may be corrugated, present a geometrical shape or a fractal design, and it may improve or decrease conductivity, for example they may be thicker or thinner, shorter or longer, and they may have different surface areas, volumes and surface/volume aspect ratios among them, that is, the working electrode (10) may be thinner or thicker than the working electrode (12) or any other electrode, and be longer or shorter or present different shapes, as long as it is adequate for the phosphate sensor (1) to work. Therefore, the size, aspect ratios and shape of the electrodes and other parts of the electrochemical sensor (1) depicted in Figure 2 have only illustrative purposes, and may vary from one embodiment to another, therefore they may be bigger or smaller, longer or shorter, or thicker or thinner than what is represented in Figure 2, as long as they present an adequate shape.

The sampling area (22) shown in Figure 2 comprise part of the working electrode (10) and part of the counter electrode (12), and is configured to receive the sample and keep it in contact with said electrodes. The reference electrode (14) in Figure 2 is protected by a layer to avoid contact with the sample, however in other embodiments of the invention it may be protected differently or be placed elsewhere. In some other embodiments of the invention the sampling area may comprise only the working electrode (12), the working (10) and counter (12) electrodes, the working (10) and counter (12) and reference electrode (14), the working (10) and counter (12) and one or more auxiliary electrodes, or the working (10),

counter (12), auxiliary and reference (14) electrodes, as long as they are configured to carry out their respective tasks and the electric circuit is closed, allowing the current to flow. It is noted that this sampling area may adopt different shapes and present different sizes, as long as it maintains the sample in contact with at least the working electrode (10), preferably with at least the working (10) and counter (12) electrodes.

The electrodes depicted in Figure 1 and/or Figure 2, preferably the working electrodes (10), may comprise cobalt in their composition, preferably in their surface to have direct contact with the phosphate of the sample. It is noted that, as demonstrated in the examples, the electrodes of the phosphate sensor (1), preferably the working electrodes (10), may comprise cobalt, nickel, tungsten, iron or any of their alloys in their composition, preferably in their surface to have direct contact with the phosphate of the sample.

It is noted that even though in the phosphate sensor (1) of Figures 1 or 2 there is not depicted a permselective membrane covering any of the electrodes, in some embodiments of the invention the phosphate sensor (1), the sampling area (22), or any of the electrodes may comprise a permselective membrane to allow the flow through said membrane of at least phosphate ions. Preferably, said permselective membrane blocks the flow through said membrane of at least a reducing agent, oxidizing agent, inorganic and/or organic species, protein and/or biomolecule. It is further noted that said permselective membrane may adopt different configurations, as long as it filters the sample before it gets in contact with at least the working electrode. Also, said permselective membrane may comprise any material fit for the task of selectively allow the fluid through said membrane, such as, but not limited to, Nafion, poly(vinyl alcohol), cellulose acetate, chitosan, ion-exchange membranes based on sulfonated polymers or polyelectrolyte multilayers, or molecularly imprinted polymers. In some other embodiments of the invention said permselective membrane blocks the flow through the membrane at least to phosphate, preferably it allows the flow to oxidizing or reducing agents and/or biomolecules.

Advantageously, the sensor of the invention is able to measure phosphate levels in blood, serum and/or plasma in a fast, reliable, simple and inexpensive way. Furthermore, the sample measured by this sensor may be undiluted and unmodified, and is based on a direct reaction that does not need enzymes or intermediate agents. The phosphate sensor of the invention employs simple electrodes, with fabrication processes well known to the skilled person, and does not require to fabricate complex electrodes or nanostructures. Also, this method does not require new or complicated materials, as it uses electrodes based on simple, available and easy to fabricate materials.

In another embodiment of the second aspect of the invention, the sensor further comprises an analysing unit configured to measure and analyse a direct redox current generated by the voltage applied between the working electrode (10) (first electrode) and the counter electrode (12) (second electrode), and/or between a reference electrode (14) and the working (first) electrode (10) wherein said direct redox current is originated at the interface between the working electrode (10) and/or the counter electrode (14), and the blood, serum and/or plasma sample (20).

It is noted that said analysing unit may comprise any of the following elements: a signal transducer to convert the electrochemical response into a measurable signal, an amplifier and signal processing circuitry, to enhance and condition the electrical signal generated by the electrochemical cell, a data acquisition and control system, to acquire, process, and store the electrochemical data, and which may include analog-to-digital converters (ADCs), microcontrollers or microprocessors, memory, information relative to the calibration curves, interfaces for data communication and control, instructions and/or software, a display or output surface, to provide visual feedback or output the measurement results, a power supply and a control and user interface to allow the user to configure the measurement parameters, start and stop measurements, and interact with the analysing unit.

Advantageously, comprising an analysing unit allows to analyse the redox current measured for a given voltage and relate it to a phosphate concentration by comparison with reference data and/or reference curves.

According to a preferred embodiment of the second aspect of the invention, at least part of the surface of the working electrode (10) of the phosphate sensor of the invention comprises a permselective membrane that allows the flow through said membrane of at least phosphate ions, wherein said permselective membrane preferably blocks the flow through said membrane of at least reducing agent, oxidizing agent, inorganic and/or organic species, protein and/or biomolecule.

In some embodiments of the invention a permselective membrane may surround any of the electrodes, preferably the working electrode (10), and this membrane may be the same or different for each electrode. In some other embodiments of the invention the permselective membrane may surround the sampling area (22) or volume comprising said area (22), or adopt any configuration that allows the sample to pass through said membrane before getting in contact with at least the working electrode (10). Said permselective membrane may comprise any material known to the skilled person adequate to carry out the task of selectively allow the flow through said membrane of the electrolytes in the sample, such as, but not limited to, Nafion, poly(vinyl alcohol), cellulose acetate, chitosan, ion-exchange membranes based on sulfonated polymers or polyelectrolyte multilayers, or molecularly imprinted polymers. In some other embodiments of the invention, the permselective membrane blocks the flow through said membrane of at least a reducing agent and/or biomolecule, or the combination of at least a reducing and at least an oxidizing agent and/or biomolecule. According to another preferred embodiment of the invention, the permselective membrane may configured to block the flow through said membrane to phosphate ions, preferably while allowing the flow through the membrane of at least a reducing agent,

oxidizing agent, inorganic and/or organic species , protein and/or biomolecule, more preferably allowing the flow through the membrane of any another reducing agent, oxidizing agent, inorganic and/or organic species molecule, protein and/or biomolecule. In another preferred embodiment of the invention, said permselective membrane may be controlling the flow of the sample only towards a reference electrode (14) comprised in the phosphate sensor of the invention, that is, it may be comprised, surrounding, protecting or covering only the reference electrode (14), to filter out the phosphate in order to obtain a reference measurement of the blood, serum and/or plasma sample.

Advantageously, the presence of the permselective membrane allows to filter out agents and/or biomolecules that may interfere with the reaction at the interface electrode/electrolyte, whereas it allows the flow of the phosphate of interest. This way, it is possible to obtain more reliable, precise and robust measurements, such that different levels of different oxidising or reducing agents may not interfere with the measurements.

According to a preferred embodiment of the invention, the sampling area (22) of the phosphate sensor (1) further comprises at least part of the surface of a reference electrode (14).

It is noted that this reference electrode may be protected, encapsulated, enclosed or separated from the sample by an impervious layer or by any other means. It is also noted that the reference electrode may be in contact with a reference solution, different from the sample and also isolated from this one. Therefore, the phosphate sensor used in the method of the invention may comprise a reference electrode. Preferably the phosphate sensor used in the method of the invention comprises a reference electrode in addition to the working electrode (10) and the counter electrode (12). Additionally, the phosphate sensor of the invention may also comprise one or more auxiliary electrodes.

Advantageously, comprising a reference electrode may play a crucial role in an electrochemical sensor by providing a stable reference potential against which the potential of the working electrode is measured. It establishes a fixed reference point for accurate and meaningful electrochemical measurements.

In another preferred embodiment of the second aspect of the invention, the phosphate sensor (1) is configured to apply a constant voltage, preferably at a voltage between -2.0 and +2.0 volts, more preferably -1.2 and 1.2 volts, even more preferably between -0.6 and +0.4 volts.

Preferably, the voltage is kept constant for an adequate amount of time to perform a current measurement, such as, but not limited to, 10 s, 5 s, 2 s, 1s, 0.5 s, 0.1 or 0.05 s. It is noted that any quantity for time or voltage aforementioned may vary by a ± 30 %, by a ± 20 %, by a ± 15 %, by a ± 10 % or by a ± 5 %. Preferably the voltage is equal or has a bigger absolute value than the open circuit potential (OCP) of the electrochemical sensor. For example, if the OCP is +0.2 V, then preferably the constant voltage applied may be +0.2 V or more, such as +0.5 V, or, if the OCP is -0.3 V, then preferably the constant voltage applied may be -0.3 V or more negative voltages, such as -0.6 V. Some examples of a measurement technique wherein the current is measured while a constant voltage is applied between the working electrode (10) and the counter electrode (12) are amperometry and chronoamperometry.

Advantageously, keeping the voltage constant for an adequate amount of time allows to obtain a measurement of the current that can be related to the amount of phosphate in the sample.

According to a preferred embodiment of the invention, the phosphate sensor (1) is configured to apply a varying voltage with time, preferably it is varied between voltages comprised in the range between -2.0 and +2.0 volts, more preferably between -1.2 and +1.2 volts, even more preferably in the range between -0.6 and +0.4 volts.

Preferably, the voltage is varied at a fixed and adequate scan rate to perform a current measurement. Preferably the voltage is varied at a scan rate between 0 and 5 volts per second, more preferably between 0 and 1 volt per second, even more preferably between 0 and 0.5 volts per second, even more preferably between 0 and 0.1 volts per second, even more preferably between 0.01 and 0.05 volts per second. It is noted that any quantity for scan rate or voltage aforementioned may vary by a ± 30 %, by a ± 20 %, by a ± 15 %, by a ± 10 % or by a ± 5 %. Some examples of a measurement technique wherein the current is measured while a varying voltage is applied between the working electrode (10) and the counter electrode (12) are voltammetry, Linear Sweep Voltammetry or Cyclic Voltammetry. It is noted that, in order to relate the measured current as a function of the applied varying voltage, the current corresponding to a particular voltage value is selected and compared with the reference data. Preferably, this voltage is the anodic or the cathodic peak potential, however, this voltage may be any other chosen voltage inside the voltage range.

Advantageously, varying the voltage at an adequate scan rate allows to obtain a measurement of the current that can be related to the amount of phosphate in the sample.

In a preferred embodiment of the second aspect of the invention, the analysing unit of the phosphate sensor (1) is further configured to measure current responses generated by the applied voltage via amperometry, preferably chronoamperometry, or voltammetry, preferably via Linear-Sweep Voltammetry, Cyclic voltammetry, Normal Pulse Voltammetry, Square-Wave Voltammetry or Differential Pulse Voltammetry, more preferably via Linear-Sweep Voltammetry or Cyclic voltammetry.

It is noted that the skilled person may know different techniques for measuring the current as a function of the applied voltage, that may be used for the method of the invention as long as there is a correlation between the measured current value and the phosphate concentration in the sample. Also, it is noted that these experimental techniques may be used alone or in combination to determine the phosphate concentration in a sample, preferably a blood, serum and/or

plasma sample, and they also may be used alone or in combination to generate the one or more calibration curves correlating a redox current versus voltage applied (varying voltage, or fixed voltage) for different concentrations of phosphate.

Advantageously, using any of these experimental techniques alone or in combination allows for the creation of calibration curves of current intensity versus voltage for different phosphate concentrations, that in turn can be used in the phosphate sensor (1) of the invention to determine the phosphate concentration in a sample, preferably a blood, serum and/or plasma sample.

In a preferred embodiment of the invention, wherein the analysing unit of the phosphate sensor (1) is configured to perform a multivariate data analysis to calculate the concentration of phosphate in the substance.

Advantageously, this allows for a quick calculation of the phosphate levels in the blood, serum and/or plasma sample.

According to another preferred embodiment of the invention, the phosphate sensor (1) further includes a data storage device or a data transmitter, preferably a wireless network transmitter.

Advantageously, this allows to quickly communicate the results to other devices such as displays, computers, phones, or control units.

In a preferred embodiment of the invention, the phosphate sensor (1) further includes signal transmitters associated with at least the working electrode (10) and the counter electrode (12), preferably associated also with a reference electrode (14), more preferably the signal transmitters include one or more of the list consisting of a signal amplifier, a low pass signal filter, a signal multiplexer, and an analogue-to-digital converter.

Advantageously, a signal transmitter allows to communicate the measured signal at the electrodes with an electronic unit.

In another preferred embodiment of the second aspect of the invention, at least the working electrode (10) comprises cobalt in its composition.

It is noted that if the phosphate sensor comprises a reference electrode (14), this reference electrode (14) may comprise cobalt in its composition as well. Any other auxiliary electrode or the counter electrode (12) may also comprise cobalt in its composition. Cobalt is an especially adequate material for the phosphate electrochemical sensor due to the phosphate assisted redox reaction that happens on its surface, and that may be represented in the following way:

$$(1) \qquad \tfrac{1}{2}\, O_2 + 2H^+ + 2e^- \rightleftharpoons H_2O$$

$$(2) \qquad Co + H_2O \rightleftharpoons Co(OH)^+ + H^+ + 2e^-$$

$$(3) \qquad 4Co(OH)^+ + 2H_2PO_4^- \rightleftharpoons Co_3(PO_4)_2 + CoO + 3H_2O + 2H^+$$

$$(4) \qquad 4Co(OH)^+ + 2HPO_4^{2-} \rightleftharpoons Co_3(PO_4)_2 + CoO + 3H_2O$$

Advantageously, cobalt has proven to be a very effective and reliable electrode material to measure phosphate concentration in a blood, serum and/or plasma sample (see figures 3 to 6), due to the redox reaction happening in the surface of the electrode comprising cobalt, preferably at the surface of the working electrode (10) and/or the reference electrode (14).

In another preferred embodiment of the invention, the detection range of the phosphate sensor (1) includes the normal values of phosphate concentration in serum, plasma or blood of humans, preferably the detection range comprises values between 0.01 to 25 mmol/L, more preferably between 0.2 to 10 mmol/L, even more preferably between 0.3 to 3 mmol/L.

Therefore, the phosphate sensor employed in the method of the invention may have a sensitivity range adequate to detect and determine the phosphate concentration in human blood, serum and/or plasma,, whether this phosphate concentration is within the range of normal phosphate concentration values in human serum, plasma or blood, or it corresponds to possible, but abnormally high or abnormally low phosphate concentration in human blood, serum and/or plasma. It is noted that any phosphate concentration aforementioned may vary by a ± 30 %, by a ± 20 %, by a ± 15 %, by a ± 10 % or by a ± 5 %.

Advantageously, having a phosphate sensor with sensitivity within the range of possible phosphate concentration values in human blood, serum or plasma allows to determine if said phosphate concentration lies within the normal values of phosphate concentration in human serum, plasma or blood (i.e., between 0.8 and 1.5 mmol/L) or if it is abnormally

high or abnormally low, which can provide useful information regarding the health status of a patient.

According to another embodiment of the second aspect of the invention, at least the working electrode (10) of the phosphate sensor (1) is screen printed, electrodeposited or comprises a piece of bulk metal, preferably the piece of bulk metal has the shape of a wire, a sheet, a plate, a disc or any other adequate bulk metal shape.

It is noted that any other electrode, such as the counter electrode (12), the reference electrode (14) or any other auxiliary electrode may also be screen printed, electrodeposited or comprises a piece of bulk metal, wherein preferably said piece of bulk metal has the shape of a wire, a sheet, a plate, a disc or any other adequate bulk metal shape. It is noted as well that the skilled person may know many different ways of fabricating electrodes, that can be applied to the phosphate sensor of the method of the invention, such as, but not limited to, interdigitated electrodes, microelectrodes, nanoelectrodes.

Advantageously, the above-mentioned shapes for the electrodes used in the phosphate sensor (1) of the invention are adequate to react with the blood, serum and/or plasma sample and to obtain a current intensity for a given voltage that, when compared with a reference curve or reference values, allows to determine the phosphate levels in the sample.

In a preferred embodiment of the invention it is disclosed a phosphate sensor (1) for determining the presence and/or concentration of phosphate in vivo in blood, serum and/or plasma of a patient, the phosphate sensor (1) comprising a sampling area (22), said sampling area (22) comprising at least part of the surface of a working electrode (10), wherein said working electrode (10) is configured to be at least partially placed intradermally and in contact with blood of the patient, wherein the phosphate sensor (1) further comprises a control unit configured to apply a voltage between the working electrode (10) and a counter electrode (12), wherein said working electrode (10) is further configured to generate a current associated to a direct redox reaction between said at least part of the working electrode (10) and the provided isolated blood, serum and/or plasma sample (20), and wherein at least the working electrode (10) comprise a metal from the list consisting of cobalt, nickel, tungsten, iron or their alloys.

It is noted that the at least part of the working electrode (10) may be comprised in a lab on a chip, a patch, or any other sensor and/or biosensor with means of at least partial intradermal placement.

Advantageously, this way the patient can have the phosphate levels monitored in real time.

In a preferred embodiment of the second aspect of the invention, the phosphate sensor (1) is multiplexed, therefore a housing comprises at least two or more sensors (1) according to the invention, allowing to reduce the noise in the measurement, increase speed and accuracy, and compare with reference samples (20) or reference electrolytes. Each of these multiplexed sensors (1) may comprise or not different permselective membranes, and/or different number and/or type of electrodes and/or comprise different materials to detect different analytes.

Advantageously, in this way, the measurement is improved in quality, quantity and reliability.

## Examples

Reagents and materials

Sodium chloride, sodium hydroxide standard solution (1 mol/L), hydrochloric acid (35%), D-(+)-glucose (≥ 99,5%), urea, bovine serum albumin (BSA), boric acid (>99,5%), potassium hydrogen phthalate (KHP), potassium hydrogen phosphate, and sodium hydrogen carbonate, were of analytical grade and purchased from Sigma-Aldrich. Ammonium molybdate (VI) tetrahydrate (99% for analysis), cobalt (II) sulfate heptahydrate (98%) and cobalt wire (0.5 mm (0.02 in) diameter, Puratronic®, 99.9995%) were purchased from ThermoScientific. Tween® 20 (synthesis grade) was purchased from Scharlab. Screen-printed electrodes (SPE) with a 4-mm working electrode, a carbon counter electrode and a Ag/AgCl pseudo-reference electrode were fabricated in-house.

Instrumentation

Potentiometric measurements were carried out at room temperature with an electromotive force (EMF) high-input impedance EMF 6 multichannel data acquisition device from Lawson laboratories, Inc. (Malvern, USA). A double-junction Ag/AgCl/KCl 3 mol/L reference electrode (type 6.0726.100, Metrohm) containing 1 mol/L of lithium acetate as bridge electrolyte was used as reference electrode. Before the addition of the target ion, the EMF for each system was first recorded in the background electrolyte until stable signal was obtained (c.a. 600 seconds). Lineal Sweep Voltammetry (LSV) experiments were performed using a PalmSens3 potentiostat (PalmSens, Netherland) with a three-electrode cell configuration. LSV were recorded in the anodic direction (i.e., from negative to positive potentials) at scan rate of 10 mV/s. Unless stated, the background electrolyte (referred as artificial serum used for electrochemical experiments had the following composition: 0.11 mol/L NaCl, 0.029 mol/L NaHCOs, $2.5\times10^{-3}$ mol/L urea, $4.7\times10^{-3}$ mol/L glucose and 50 g/L BSA, and adjusted at pH 7.4. Phosphate stock solutions were prepared by dissolving appropriate amounts of $KH_2PO_4$ in the background electrolyte. Spectrophotometric assays were carried out on spectrophotometer UV/VIS 4000 from Dinko

Instruments (Barcelona, Spain)

Spectrophotometric measurements

The colorimetric assay PHOS2 developed by Roche was employed as the reference method to validate the usage of Co-based electrodes for the determination of phosphate ions in biological samples. The PHOS2 assay is used on Roche/Hitachi Cobas C analysers to measure the levels of phosphate ions in human serum and/or plasma, and urine. The analytical procedure was performed according to the PHOS2 booklet instructions.

Sample preparation

Blood samples were extracted by venipuncture procedure from healthy volunteers and were collected in tubes containing sodium heparin (green-cap tubes). Plasma was separated from blood cells by centrifuge at 2000 g for 10 minutes. Plasma samples with different levels of phosphate were prepared by spiking the sample with different amounts of a standard solution of sodium hydrogen phosphate. All samples were stored at 4 °C for no more than one week.

**Example 1**

Cobalt wires (0.5 mm (0,02 in) diameter, Puratronic®, 99.9995%) cut in pieces of two centimeters were used as working electrodes. A platinum wire and Ag/AgCl(sat) were used as counter and reference electrodes, respectively. Calibration curves were obtained by recording LSV between -0.8 to +0.2 V after successive additions of the phosphate stock solutions to the background electrolyte. The concentration of phosphate ions was varied from $1\times10^{-4}$ to $1\times10^{-2}$ mol/L (from -4 to -2 on a logarithmic scale). The current values obtained when -0.4, -0.3 and -0.2 V were applied during the LSV scan were used as analytical response toward phosphate ions, as is shown in Figure 3.

**Example 2**

Carbon-based SPE modified with electrodeposited cobalt films were used as working electrodes. Cobalt films were electrodeposited on SPE by applying a fixed potential of -1.1 V (vs Ag/AgCl pseudo reference electrode) for 180 seconds. The electrolytic bath consisted of 1.1 mol/L $CoSO_4$, 0.022 mol/L $H_3BO_3$, and 0.051 mol/L NaCl adjusted at pH 5.1 After the cobalt deposition, electrodes were washed with Milli-Q water and ethanol and dried at room temperature. Human plasma was spiked with phosphate ions stock solution to obtain plasma samples with phosphate levels between $1.2\times10^{-3}$ and $6.7\times10^{-3}$ mol/L. A calibration curve was obtained by recording LSV between -0.8 to +0.2 V using one electrode per each standard and selecting the current when - 0.3 V was applied in the LSV scan as analytical response toward phosphate concentrations, as is shown in Figure 4. Estimation of phosphate concentration in spiked samples was performed by interpolating the measured current by LSV in the calibration curve. The concentrations of phosphate ions obtained by interpolation on the calibration curve were compared with those using the gold standard method (PHOS2 assay from Roche) for the determination of phosphate ions in human fluid samples.

**Example 3**

SPE modified with cobalt-based inks were used as working electrodes. Cobalt-based inks were prepared by mixing:

- Cobalt powder (<150 μm, ≥99.9% trace metals basis, Merck) with commercial carbon ink (C2030519P4, from Gwent Group) at 30:70 and 60:40% w/w, referred in the figure as 30%Co+70% Carbon ink and 60%Co+40% Carbon ink, respectively.

- Cobalt powder, graphite powder and nail polish at 65:9:26% w/w, referred in the figure as Nail polish+Co+Graphite.

The cobalt-based inks were printed on SPE using a semi-automatic screen-printing machine. Calibration curves were obtained by recording LSV between -0.8 to +0.5 V after successive additions of the phosphate stock solutions to the background electrolyte. Currents obtained when +0.5 V was applied during LSV scan were used as analytical response toward phosphate ions, as is shown in Figure 5.

**Example 4**

SPE modified with electrodeposited FeCo alloy films were used as working electrodes. FeCo films were

electrodeposited on SPE by applying a fixed potential of -1.2 V (vs Ag/AgCl pseudo reference electrode) for 35 seconds. The electrolytic bath consisted of 0.2 mol/L CoSO4·, 0.166 mol/L Mohr salt, 0.041 mol/L $H_3BO_3$, and 0.51 mol/L NaCl adjusted at pH 2.1 After the FeCo film deposition, electrodes were washed with Milli-Q water and ethanol and dried at room temperature. Artificial serum was spiked with phosphate ions stock solution to obtain standards with phosphate levels between $0.25\times10^{-3}$ and $8\times10^{-3}$ mol/L. A calibration curve was obtained by recording LSV between -0.8 to +0.2 V using one electrode per each standard and selecting the current when - 0.2 V was applied during the LSV scan as analytical response toward phosphate concentrations, as is shown in Figure 6.

**Example 5**

SPE modified with electrodeposited Ni films were used as working electrodes. Ni films were electrodeposited on SPE by applying a fixed potential of -1.2 V (vs Ag/AgCl pseudo reference electrode) for 90 seconds. The electrolytic bath consisted of 1.1 mol/L NiSOa, 0.49 mol/L $H_3BO_3$, and 0.14 mol/L NaCl adjusted at pH 5.2. After the Ni film deposition, electrodes were washed with Milli-Q water and ethanol and dried at room temperature. Artificial serum was spiked with phosphate ions stock solution to obtain standards with phosphate levels between $0.25\times10^{-3}$ and $8\times10^{-3}$ mol/L. A calibration curve was obtained by recording LSV between -0.8 to +0.4 V using one electrode per each standard and selecting the current when -0.4 V was applied during the LSV scan as analytical response toward phosphate concentrations, as is shown in Figure 7.

**Example 6**

Tungsten foil (0.25 mm thick, 99.95% from Alfa Aesar) cut in pieces of 2.0x0.5 centimeters (L$\times$W) were used as working electrodes. A platinum wire and Ag/AgCl(sat) were used as counter and reference electrodes, respectively. Calibration curves were obtained by recording cyclic voltammetry between -0.7 to +1.3 V at scan rate of 25 mV/s after successive additions of the phosphate stock solutions to the background electrolyte. The current values obtained when +0.8 V was applied during the CV scan were selected as analytical response toward phosphate concentrations, as is shown in Figure 8. The concentration of phosphate ions was varied from $0.25\times10^{-3}$ to $8\times10^{-3}$ mol/L.

**Example 7**

SPE modified with electrodeposited cobalt films were used as working electrodes. Cobalt films were electrodeposited on SPE by applying a fixed potential of -1.1 V (vs Ag/AgCl pseudo reference electrode) for 180 seconds. The electrolytic bath consisted of 1.1 mol/L $CoSO_4$, 0.022 mol/L $H_3BO_3$, and 0.051 mol/L NaCl adjusted at pH 5.1 After the cobalt deposition, electrodes were washed with Milli-Q water and ethanol and dried at room temperature. Four human plasma samples with known phosphate concentrations were used as standards to construct the calibration curve. An aliquot of 40 $\mu$L of each standard was poured over the SPE and then, a LSV between -0.8 to +0.2 V was recorded. The current obtained for each standard when -0.4 V was applied in the LSV scan was used to construct the calibration plot, as is shown in Figure 9. A similar procedure was used to measure the four plasma samples and the obtained current was interpolated in the calibration plot as depicted in Figure 9.

**Example 8**

Human plasma was spiked with different amounts of hydrogen phosphate ions to obtain samples with different levels of this ion. The resulting phosphate concentration was determined following the PHOS2 colorimetric assay. These samples were subject to further analysis via potentiometry, employing cobalt wires (0.5 mm (0.02 in) diameter and 20 mm in length) as indicator electrodes . The potentiometric analysis entailed immersing the cobalt electrodes in $25\times10^{-3}$ mol/L KHP buffer solution, followed by the sequential addition of hydrogen phosphate ion to obtain a final concentration of $1\times10^{-4}$ and $1\times10^{-2}$ mol/L. After recording the EMF at these two concentration points, the electrodes were then submerged in 1:10 diluted samples, and the resulting potential was measured. The obtained potential values were subsequently utilized to estimate the phosphate concentration within each sample, using an interpolation method based on a two-point calibration curve developed from the measurements at $1\times10^{-4}$ mol/L and $1\times10^{-2}$ mol/L of hydrogen phosphate solutions, as is shown in Figure 10.

**CLAUSES**

1. A method for determining the presence and/or concentration of phosphate in an isolated bodily fluid sample (20),

preferably blood, serum and/or plasma sample (20), the method comprising;

a. providing the isolated bodily fluid sample (20) preferably blood, serum and/or plasma sample (20) in a sampling area (22), the sampling area (22) comprising at least part of the surface of a first electrode (10),

and the method further comprising either one of the following steps:

b. applying a voltage between the first electrode (10) and a second electrode (12) and measuring a current between said first (10) and second (12) electrodes, or,

c. measuring a voltage between the first electrode (10) and the second electrode (12),

wherein the presence and/or concentration of phosphate in the isolated bodily fluid sample (20), preferably blood, serum and/or plasma sample (20), is determined by the current or the voltage measured between the first electrode (10) and the second electrode (12), wherein at least the first electrode (10) is characterized by comprising a metal from the list consisting of cobalt, nickel, tungsten, iron or any of their alloys, preferably cobalt, and at least part of the first electrode (10) is further configured to generate a current associated to a direct redox reaction between said at least part of the first electrode (10) and the provided isolated bodily fluid sample (20), preferably blood, serum and/or plasma sample (20), and wherein if step c is performed, prior to said step of measuring the voltage between the first electrode (10) and the second electrode (12), the pH of the isolated bodily fluid sample, preferably blood, serum and/or plasma, is reduced below 7.4, preferably between 1 and 7, more preferably between 3.4 and 5.4.

2. The method for determining the presence and/or concentration of phosphate in an isolated blood, serum and/or plasma sample (20) according to claim 1, the method comprising:

a. providing the isolated blood, serum and/or plasma sample (20) in a sampling area (22), the sampling area (22) comprising at least part of the surface of a first electrode (10),

b. applying a voltage between the first electrode (10) and a second electrode (12),

c. subsequently or simultaneously to step b, measuring a current between the first electrode (10) and the second electrode (12),

wherein the presence and/or concentration of phosphate in the isolated blood, serum and/or plasma sample (20) is determined by the measured current between the first electrode (10) and the second electrode (12), and wherein at least the first electrode (10) is characterized by comprising a metal from the list consisting of cobalt, nickel, tungsten, iron or any of their alloys, preferably cobalt, and at least part of the first electrode (10) is further configured to generate a current associated to a direct redox reaction between said at least part of the first electrode (10) and the provided isolated blood, serum and/or plasma sample (20).

3. The method according to claim 1 or 2, wherein at least part of the surface of the first electrode (10) comprises a permselective membrane that allows the flow through said membrane of at least phosphate ions, wherein said permselective membrane preferably blocks the flow through said membrane of at least a reducing agent, an oxidizing agent, inorganic and/or organic species, protein and/or biomolecule.

4. The method according to claim 2 or 3, wherein the determination of the presence and/or concentration of phosphate in the isolated blood, serum and/or plasma sample (20) is performed by comparing the value of the current measured with reference values of current versus phosphate concentration, preferably the reference values are obtained from a dependency curve between the current and the concentration of phosphate.

5. The method according to any of claims 2 to 4, wherein the sampling area (22) further comprises at least part of the surface of a reference electrode (14).

6. The method according to any of claims 2 to 5, wherein the applied voltage is kept constant, preferably at a voltage between -1.2 and +1.2 volts, more preferably between -0.6 and +0.4 volts.

7. The method according to any of claims 2 to 5, wherein the applied voltage is varied with time, preferably is varied between voltages comprised in the range between -1.2 and +1.2 volts, more preferably in the range between -0.6 and

+0.4 volts.

8. The method according to any of claims 2 to 7, wherein the measured current between the first electrode (10) and the second electrode (12) under applied voltage is measured via amperometry, preferably chronoamperometry, or via voltammetry, preferably Linear-Sweep Voltammetry, Cyclic voltammetry, Normal Pulse Voltammetry, Square-Wave Voltammetry or Differential Pulse Voltammetry, more preferably via Linear-Sweep Voltammetry or Cyclic voltammetry.

9. The method according to any of claims 1 to 8, wherein at least the first electrode (10) comprises cobalt in its composition.

10. The method according to any of claims 1 to 9, wherein the determined concentration of phosphate comprises values within the possible values of phosphate concentration in human serum/plasma or blood, preferably the concentration of phosphate comprises values between 0.01 to 25 mmol/L, more preferably between 0.2 to 10 mmol/L, even more preferably between 0.3 to 3 mmol/L.

11. The method according to any of claims 1 to 10, wherein at least the first electrode (10) is screen printed, electrodeposited or comprises a piece of bulk metal, preferably the piece of bulk metal has the shape of a wire, a sheet, a plate, a disc or any other adequate bulk metal shape.

12. A phosphate sensor (1) for determining the presence and/or concentration of phosphate in a blood, serum and/or plasma sample (20), the phosphate sensor (1) comprising a sampling area (22), said sampling area (22) comprising at least part of the surface of a first electrode (10), wherein said first electrode (10) is configured to be at least partially in contact with the blood, serum and/or plasma sample (20) provided in the sampling area (22), wherein said first electrode (10) is further configured to generate a current associated to a direct redox reaction between said at least part of the first electrode (10) and the provided isolated blood, serum and/or plasma sample (20), and wherein at least the first electrode (10) comprise a metal from the list consisting of cobalt, nickel, tungsten, iron or their alloys, preferably cobalt.

13. The phosphate sensor (12) according to claim 12, wherein the phosphate sensor (1) further comprises a control unit configured to apply a voltage between the first electrode (10) and a second electrode (12).

14. The phosphate sensor (1) according to claim 13, further comprising an analysing unit configured to measure and analyse a direct redox current generated by the voltage applied between the first electrode (10) and the second electrode (12), and/or between a reference electrode (14) and the first electrode (10), wherein said direct redox current is originated at the interface between the first electrode (10) and/or the reference electrode (14), and the blood, serum and/or plasma sample (20).

15. The phosphate sensor (1) according to claims 12 to 14, wherein at least part of the surface of the first electrode (10) comprises a permselective membrane that allows the flow through said membrane of at least phosphate ions, wherein said permselective membrane preferably blocks the flow through said membrane of at least a reducing agent, oxidizing agent, inorganic and/or organic species, protein and/or biomolecule.

16. The phosphate sensor (1) according to any of claims 12 to 15, wherein the sampling area (22) further comprises at least part of the surface of a reference electrode (14).

17. The phosphate sensor (1) according to any of claims 13 to 16, wherein the control unit of the phosphate sensor (1) is configured to apply a constant voltage, preferably at a voltage between -2.0 and +2.0 volts, more preferably -1.2 and +1.2 volts, even more preferably between -0.6 and +0.4 volts.

18. The phosphate sensor (1) according to any of claims 13 to 16, wherein the control unit of the phosphate sensor (1) is configured to apply a varying voltage with time, preferably is varied between voltages comprised in the range between -1.2 and +1.2 volts, more preferably in the range between -0.6 and +0.4 volts.

19. The phosphate sensor (1) according to any of claims 14 to 18, wherein the analysing unit is further configured to measure current responses generated by the applied voltage via amperometry, preferably chronoamperometry, or voltammetry, preferably via Linear-Sweep Voltammetry, Cyclic Voltammetry, Normal Pulse Voltammetry, Square-Wave Voltammetry or Differential Pulse Voltammetry, more preferably via Linear-Sweep Voltammetry or Cyclic

voltammetry.

20. The phosphate sensor (1) according to any of claims 14 to 19, wherein the analysing unit is configured to perform a multivariate data analysis to calculate the concentration of phosphate in the substance.

21. The phosphate sensor (1) according to any of claims 12 to 20, wherein the phosphate sensor (1) further includes a data storage device or a data transmitter, preferably a wireless network transmitter.

22. The phosphate sensor (1) according to any of claims 12 to 21, wherein the phosphate sensor (1) further includes signal transmitters associated with at least the first electrode (10) and the second electrode (12), preferably the signal transmitters include one or more of the list consisting of a signal amplifier, a low pass signal filter, a signal multiplexer, and an analogue-to-digital converter.

23. The phosphate sensor (1) according to any of claims 12 to 22, wherein at least the first electrode (10) comprises cobalt in its composition.

24. The phosphate sensor (1) according to any of claims 12 to 23, wherein the detection range of the phosphate sensor (1) includes the normal values of phosphate concentration in serum plasma or blood of humans, preferably the detection range comprises values between 0.01 to 20 mmol/L, more preferably between 0.2 to 10 mmol/L, even more preferably between 0.3 to 3 mmol/L.

25. The phosphate sensor (1) of any of claims 12 to 24, wherein at least the first electrode (10) is screen printed, electrodeposited or comprises a piece of bulk metal, preferably the piece of bulk metal has the shape of a wire, a sheet, a plate, a disc or any other adequate bulk metal shape.

## Claims

**1.** A method for determining the presence and/or concentration of phosphate in an isolated blood, serum and/or plasma sample (20), the method comprising;

a. providing the isolated blood, serum and/or plasma sample (20) in a sampling area (22), the sampling area (22) comprising at least part of the surface of a first electrode (10), preferably the sampling area (22) also comprising at least part of the surface of a second electrode (12),

and the method further comprising either one of the following steps:

b. applying a voltage between the first electrode (10) and a second electrode (12) and measuring a current between said first (10) and second (12) electrodes, or,
c. measuring a voltage between the first electrode (10) and the second electrode (12),

wherein the presence and/or concentration of phosphate in the isolated blood, serum and/or plasma sample (20) is determined by the current or the voltage measured between the first electrode (10) and the second electrode (12), wherein at least the first electrode (10) is **characterized by** comprising a metal from the list consisting of cobalt, nickel, tungsten, iron or any of their alloys, preferably cobalt, and at least part of the first electrode (10) is further configured to generate a current associated to a direct redox reaction between said at least part of the first electrode (10) and the provided isolated blood, serum and/or plasma sample (20), and wherein if step c is performed, prior to prior to said step of measuring the voltage between the first electrode (10) and the second electrode (12), the pH of the isolated blood, serum and/or plasma sample (20) is reduced below 7.4, preferably between 1 and 7, more preferably between 3.4 and 5.4.

**2.** The method for determining the presence and/or concentration of phosphate in an isolated blood, serum and/or plasma sample (20) according to claim 1, further involving a reference electrode (14), wherein the voltage of step (b) is applied between the first electrode (10) and the reference electrode (14), and the voltage of step (c) is measured between the first electrode (10) and the reference electrode (14).

**3.** The method for determining the presence and/or concentration of phosphate in an isolated blood, serum and/or plasma sample (20) according to claim 1 or 2, the method comprising:

a. providing the isolated blood, serum and/or plasma sample (20) in a sampling area (22), the sampling area (22) comprising at least part of the surface of a first electrode (10),
b. applying a voltage between the first electrode (10) and a second electrode (12), or between the first electrode (10) and the reference electrode (14), and
c. subsequently or simultaneously to step b, measuring a current between the first electrode (10) and the second electrode (12),

wherein the presence and/or concentration of phosphate in the isolated blood, serum and/or plasma sample (20) is determined by the measured current between the first electrode (10) and the second electrode (12), and wherein at least the first electrode (10) is **characterized by** comprising a metal from the list consisting of cobalt, nickel, tungsten, iron or any of their alloys, preferably cobalt, and at least part of the first electrode (10) is further configured to generate a current associated to a direct redox reaction between said at least part of the first electrode (10) and the provided isolated blood, serum and/or plasma sample (20).

**4.** The method according to any of the previous claims wherein at least part of the surface of the first electrode (10) comprises a permselective membrane that allows the flow through said membrane of at least phosphate ions, wherein said permselective membrane preferably blocks the flow through said membrane of at least a reducing agent, oxidizing agent, inorganic and/or organic species, protein and/or biomolecule.

**5.** The method according to any of claims 2 to 4, wherein the determination of the presence and/or concentration of phosphate in the isolated blood, serum and/or plasma sample (20) is performed by comparing the value of the current measured with reference values of current versus phosphate concentration, preferably the reference values are obtained from a dependency curve between the current and the concentration of phosphate.

**6.** The method according to any of claims 3 to 5, wherein the measured current between the first electrode (10) and the second electrode (12) under applied voltage is measured via amperometry, preferably chronoamperometry, or via voltammetry, preferably Linear-Sweep Voltammetry, Cyclic Voltammetry, Normal Pulse Voltammetry, Square-Wave Voltammetry or Differential Pulse Voltammetry, more preferably via Linear-Sweep Voltammetry or Cyclic Voltammetry.

**7.** A phosphate sensor (1) for determining the presence and/or concentration of phosphate in a blood, serum and/or plasma sample (20), the phosphate sensor (1) comprising a first (10) and a second (12) electrodes, preferably further comprising a reference electrode (14), and wherein the phosphate sensor further comprises a sampling area (22), said sampling area (22) comprising at least part of the surface of a first electrode (10), wherein said first electrode (10) is configured to be at least partially in contact with the blood, serum and/or plasma sample (20) provided in the sampling area (22), wherein said first electrode (10) is further configured to generate a current associated to a direct redox reaction between said at least part of the first electrode (10) and the provided isolated blood, serum and/or plasma sample (20), and wherein at least the first electrode (10) comprise a metal from the list consisting of cobalt, nickel, tungsten, iron or their alloys, preferably cobalt.

**8.** The phosphate sensor (1) according to claim 7, wherein the phosphate sensor (1) further comprises a control unit configured to apply a voltage between the first electrode (10) and the second electrode (12), or between the first electrode (10) and the reference electrode (14)

**9.** The phosphate sensor (1) according to claim 8, further comprising an analysing unit configured to measure and analyse a direct redox current generated by the voltage applied between the first electrode (10) and the second electrode (12), and/or between the reference electrode (14) and the first electrode (10), wherein said direct redox current is originated at the interface between the first electrode (10) and/or the second electrode (14), and the blood, serum and/or plasma sample (20).

**10.** The phosphate sensor (1) according to claims 7 to 9, wherein at least part of the surface of the first electrode (10) comprises a permselective membrane that allows the flow through said membrane of at least phosphate ions, wherein said permselective membrane preferably blocks the flow through said membrane of at least a reducing agent, an oxidizing agent, inorganic and/or organic species, a protein and/or a biomolecule.

**11.** The phosphate sensor (1) according to any of claims 8 to 10, wherein the control unit of the phosphate sensor (1) is configured to apply a constant voltage, preferably at a voltage between -2.0 and +2.0 volts, more preferably -1.2 and +1.2 volts, even more preferably between -0.6 and +0.4 volts.

**12.** The phosphate sensor (1) according to any of claims 8 to 11, wherein the control unit of the phosphate sensor (1) is configured to apply a varying voltage with time, preferably it is varied between voltages comprised in the range between -2.0 and +2.0 volts, more preferably -1.2 and +1.2 volts, even more preferably between -0.6 and +0.4 volts.

**13.** The phosphate sensor (1) according to any of claims 9 to 12, wherein the analysing unit is further configured to measure current responses generated by the applied voltage via amperometry, preferably chronoamperometry, or voltammetry, preferably via Linear-Sweep Voltammetry, Cyclic Voltammetry, Normal Pulse Voltammetry, Square-Wave Voltammetry or Differential Pulse Voltammetry, more preferably via Linear-Sweep Voltammetry or Cyclic voltammetry.

**14.** The phosphate sensor (1) according to any of claims 7 to 13, wherein the detection range of the phosphate sensor (1) includes the normal values of phosphate concentration in serum, plasma and/or blood of humans, preferably the detection range comprises values between 0.01 to 25 mmol/L, more preferably between 0.2 to 10 mmol/L, even more preferably between 0.3 to 3 mmol/L.

**15.** The phosphate sensor (1) of any of claims 7 to 14, wherein at least the first electrode (10) is screen printed, electrodeposited or comprises a piece of bulk metal, preferably the piece of bulk metal has the shape of a wire, a sheet, a plate, a disc or any other adequate bulk metal shape.

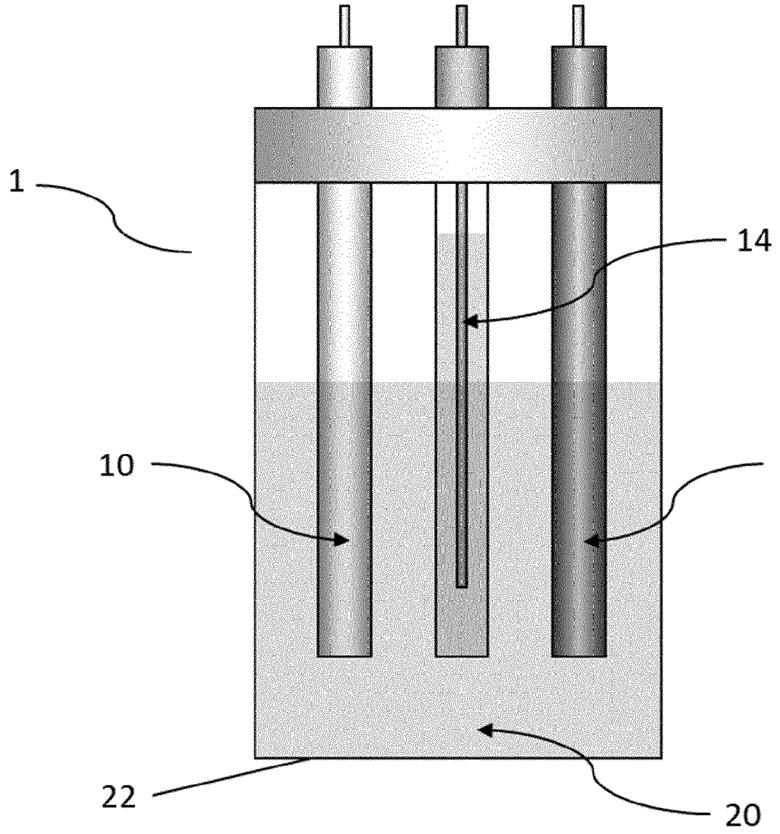


Fig. 1

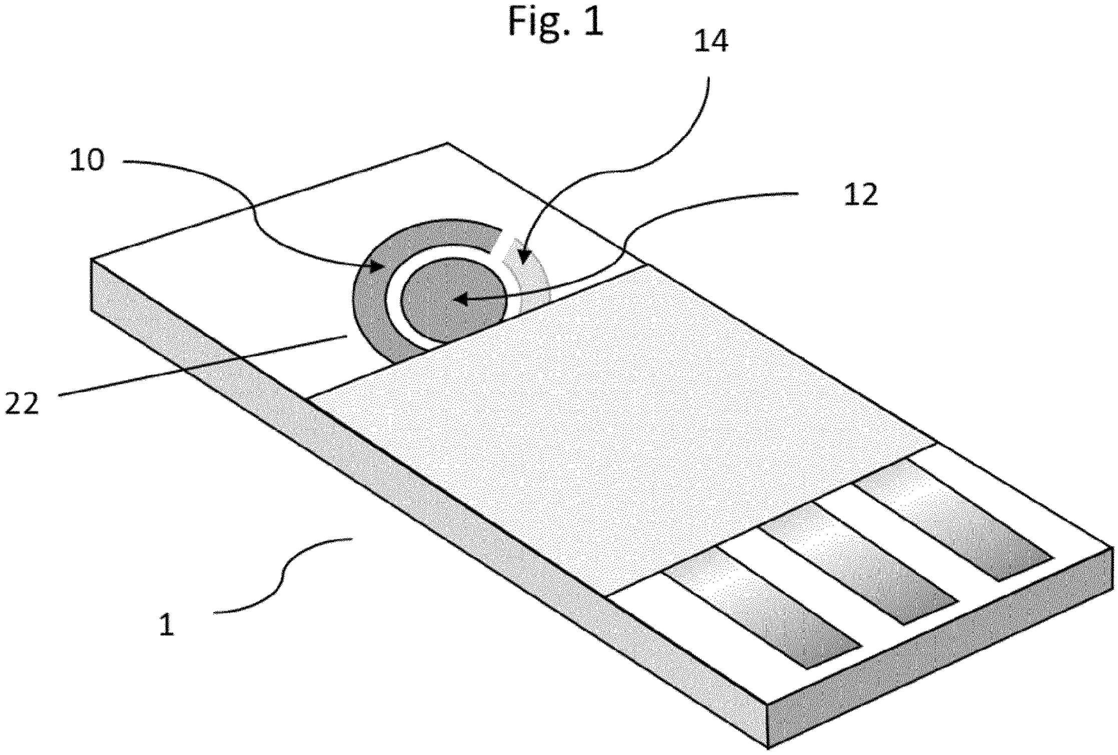


Fig. 2

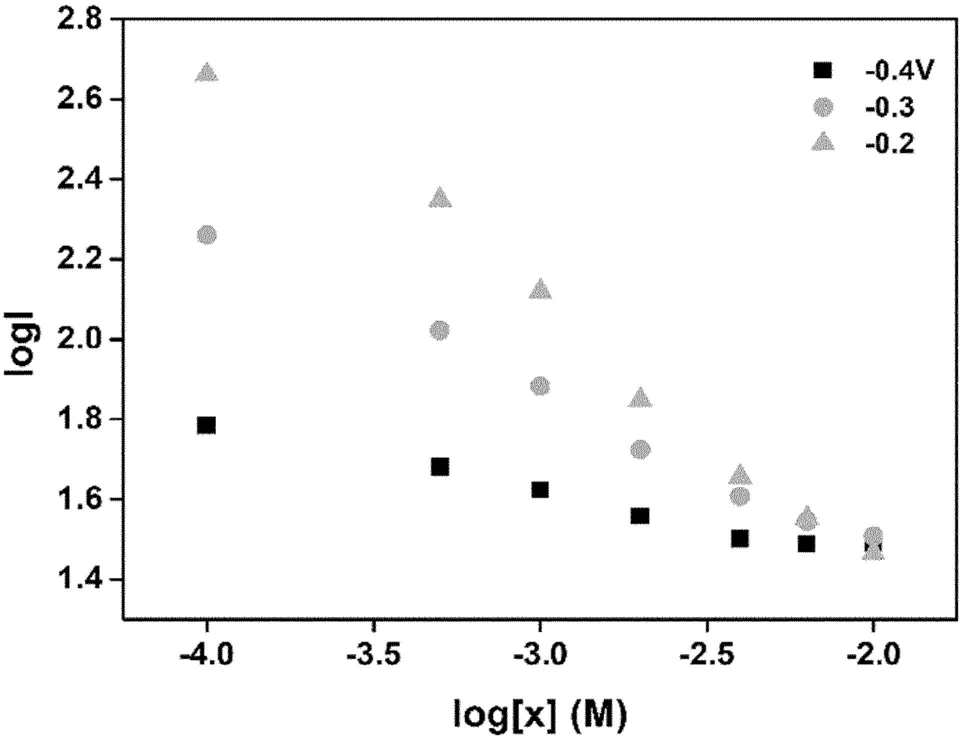
-0.4V
-0.3
-0.2
logI
log[x] (M)
2.8
2.6
2.4
2.2
2.0
1.8
1.6
1.4
-4.0
-3.5
-3.0
-2.5
-2.0

Fig. 3

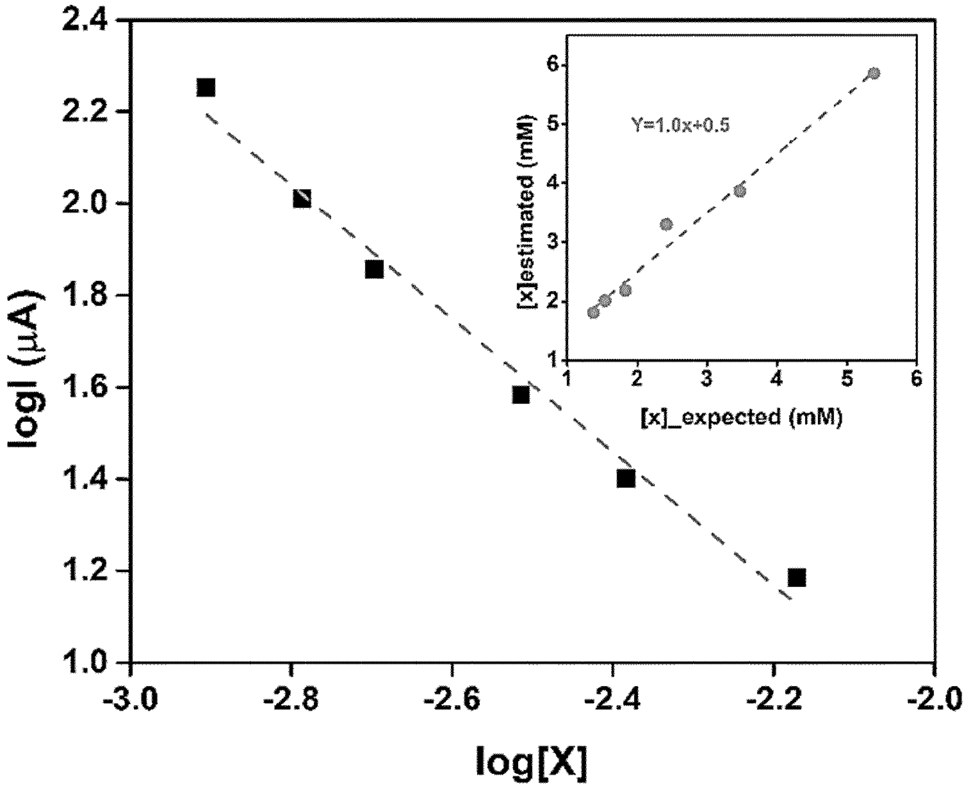
logI (μA)
log[X]
2.4
2.2
2.0
1.8
1.6
1.4
1.2
1.0
-3.0
-2.8
-2.6
-2.4
-2.2
-2.0
Y=1.0x+0.5
[x]estimated (mM)
[x]_expected (mM)

Fig. 4

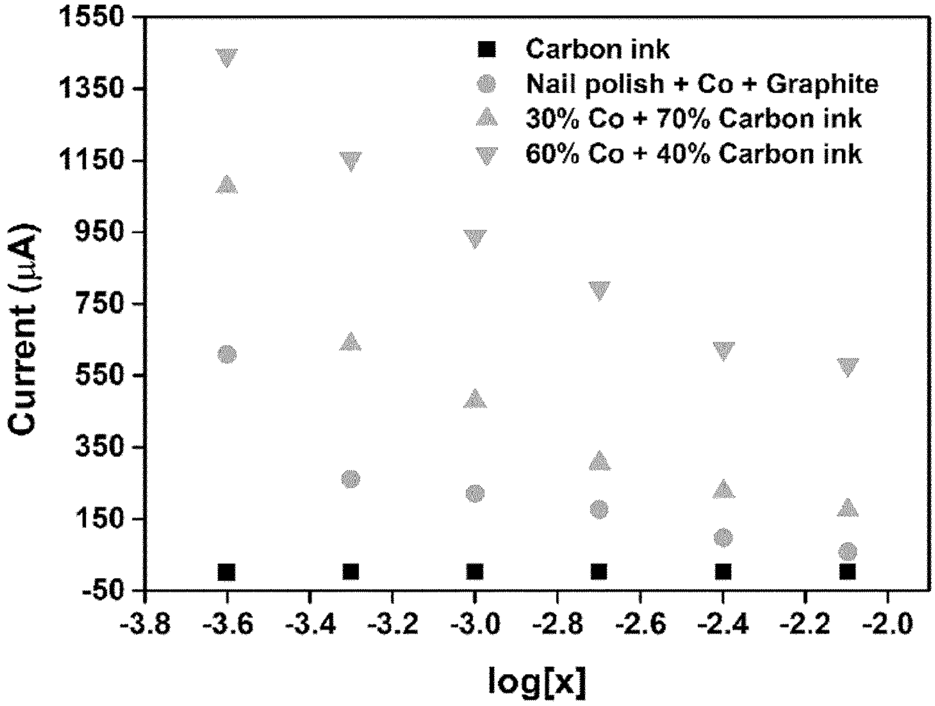

Carbon ink
Nail polish + Co + Graphite
30% Co + 70% Carbon ink
60% Co + 40% Carbon ink
Current (μA)
1550
1350
1150
950
750
550
350
150
-50
-3.8
-3.6
-3.4
-3.2
-3.0
-2.8
-2.6
-2.4
-2.2
-2.0
log[x]


Fig. 5

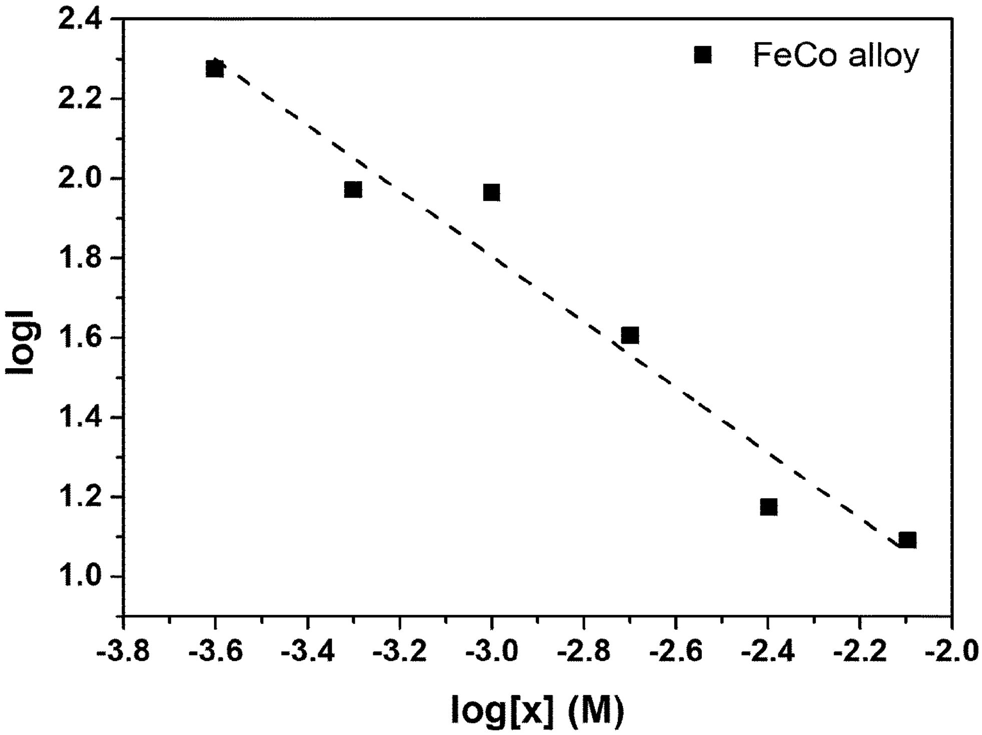

FeCo alloy
logI
2.4
2.2
2.0
1.8
1.6
1.4
1.2
1.0
-3.8
-3.6
-3.4
-3.2
-3.0
-2.8
-2.6
-2.4
-2.2
-2.0
log[x] (M)


Fig. 6

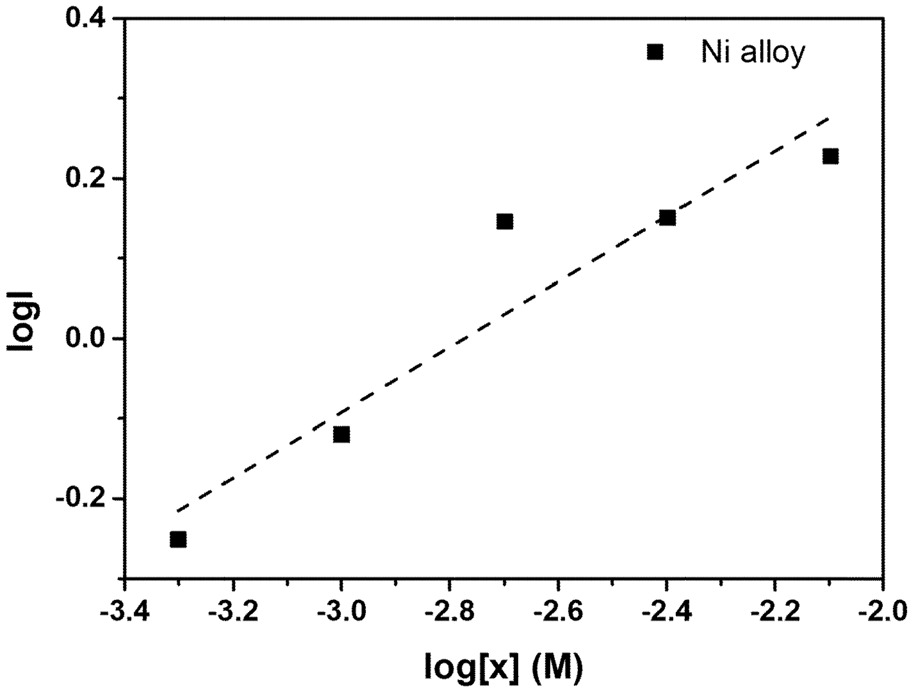
Ni alloy
0.4
0.2
0.0
-0.2
logI
-3.4
-3.2
-3.0
-2.8
-2.6
-2.4
-2.2
-2.0
log[x] (M)

Fig. 7

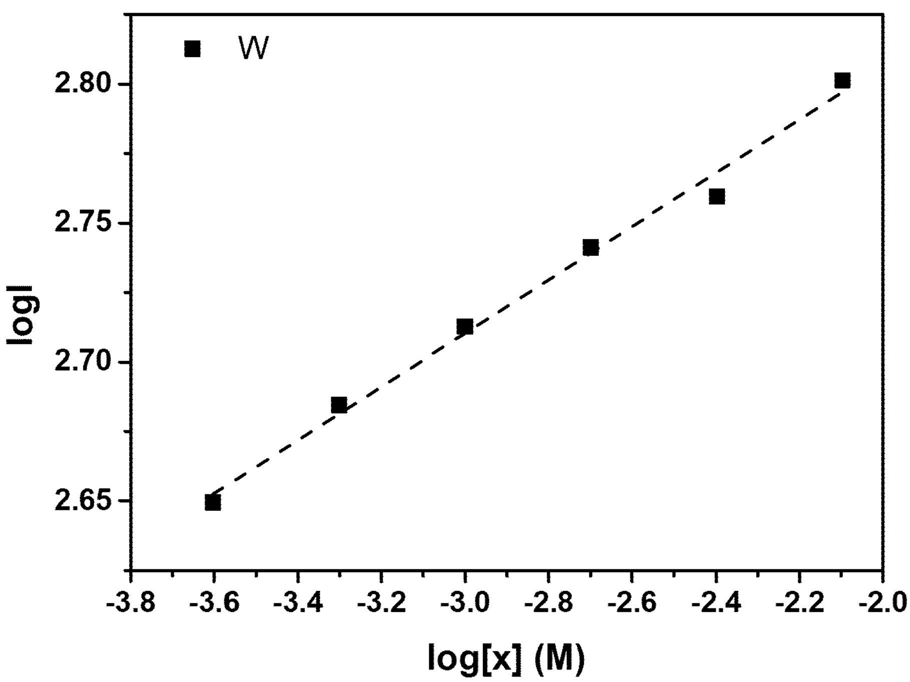
W
2.80
2.75
2.70
2.65
logI
-3.8
-3.6
-3.4
-3.2
-3.0
-2.8
-2.6
-2.4
-2.2
-2.0
log[x] (M)

Fig. 8

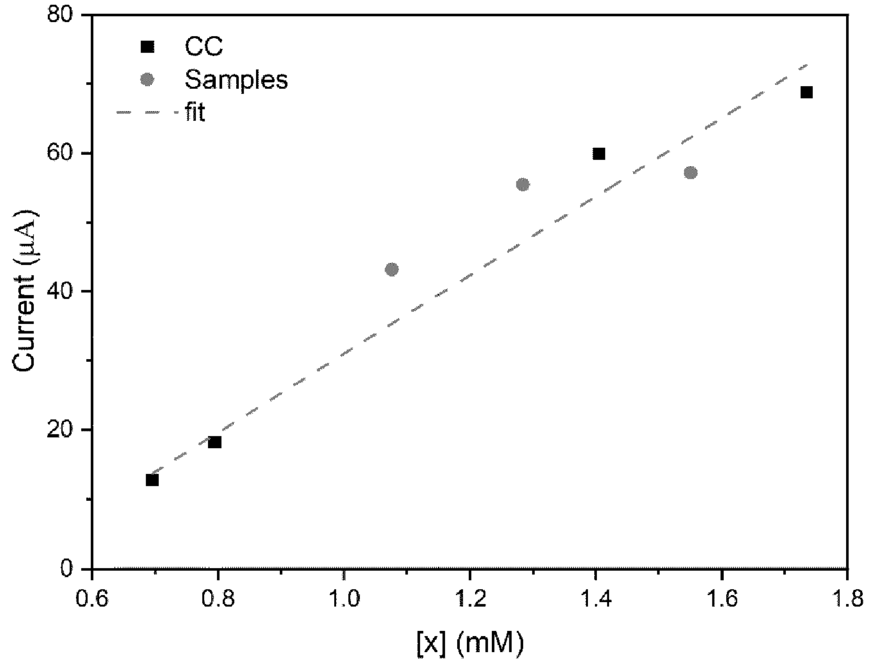
CC
Samples
fit
Current (μA)
[x] (mM)
0
20
40
60
80
0.6
0.8
1.0
1.2
1.4
1.6
1.8

Fig. 9

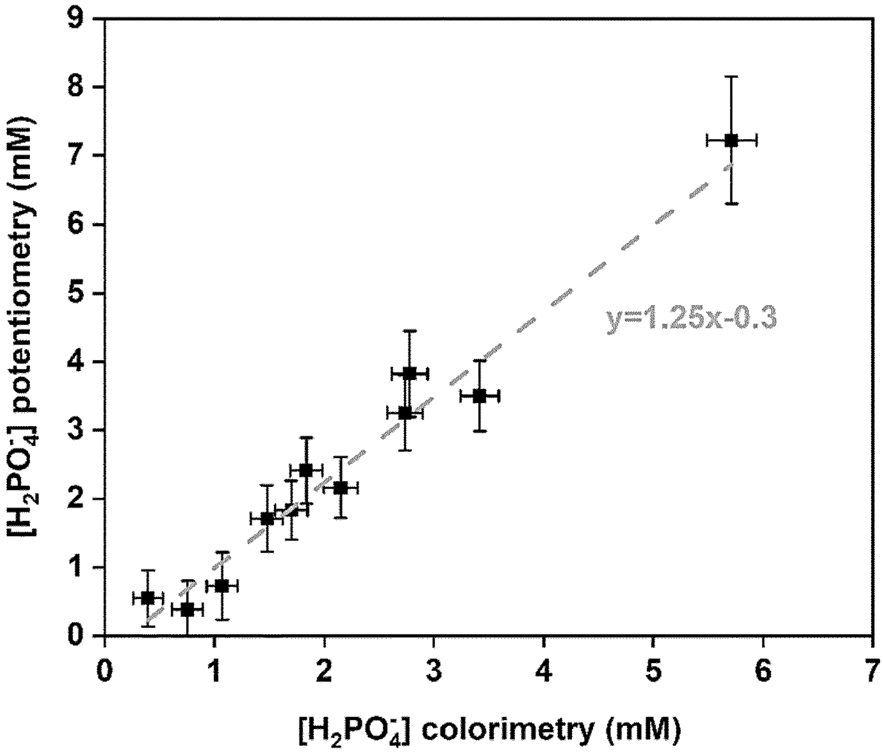
y=1.25x-0.3
[$H_2PO_4^-$] potentiometry (mM)
[$H_2PO_4^-$] colorimetry (mM)
0
1
2
3
4
5
6
7
8
9

Fig. 10

Europäisches Patentamt
European Patent Office
Office européen des brevets

# EUROPEAN SEARCH REPORT

Application Number
EP 24 38 2358

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LEI SONG ET AL: "A disposable cobalt-based phosphate sensor based on screen printing technology", SCIENCE CHINA CHEMISTRY; THE FRONTIERS OF CHEMICAL BIOLOGY AND SYNTHESIS, vol. 57, no. 9, 31 July 2014 (2014-07-31), pages 1283-1290, XP055577399, Sience China Press ISSN: 1674-7291, DOI: 10.1007/s11426-014-5127-6 | 1,7-9, 14,15 | INV. G01N27/49<br>ADD. G01N27/333 |
| Y | * abstract *<br>* 2.2 Apparatus *<br>* 2.3 Design and fabrication of phosphate sensor *<br>* figure 1 *<br>* figure 2 *<br>* 3.1 EMF response characteristics *<br>* figure 3 *<br>* 4 Conclusions *<br>-----<br>-/-- | 10 | |

TECHNICAL FIELDS SEARCHED (IPC)

G01N

The present search report has been drawn up for all claims

2

| Place of search | Date of completion of the search | Examiner |
|---|---|---|
| Munich | 2 October 2024 | Knoll, Stephan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 24 38 2358

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | XU KEBIN ET AL: "Phosphate ion sensor using a cobalt phosphate coated cobalt electrode", ELECTROCHIMICA ACTA, vol. 282, 1 August 2018 (2018-08-01), pages 242-246, XP055981303, AMSTERDAM, NL ISSN: 0013-4686, DOI: 10.1016/j.electacta.2018.06.021 | 1-3,5-9, 11-13,15 | |
| Y | * abstract * * 2.2 Apparatus * * 2.3 Preparation of Co modified electrodes * * 2.4 Electrochemical measurement * * 3.1 Cyclic voltammograms of Co electrode in phosphate ion system * * figure 1 * * figure 2 * * 3.4 Response characteristics to phosphate ion of Co electrode * * figure 5 * ----- | 4,10 | TECHNICAL FIELDS SEARCHED (IPC) |
| X | WO 2021/050867 A1 (UNIV FLORIDA [US]) 18 March 2021 (2021-03-18) * figure 1 * * paragraph [0004] - paragraph [0008] * * figure 4A * * paragraph [0025] - paragraph [0029] * ----- -/-- | 7-9,14, 15 | |

The present search report has been drawn up for all claims

2

| Place of search | Date of completion of the search | Examiner |
|---|---|---|
| Munich | 2 October 2024 | Knoll, Stephan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 24 38 2358

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SHEELA BERCHMANS ET AL: "Determination of inorganic phosphate by electroanalytical methods: A review", ANALYTICA CHIMICA ACTA, ELSEVIER, AMSTERDAM, NL, vol. 729, 29 March 2012 (2012-03-29), pages 7-20, XP028487307, ISSN: 0003-2670, DOI: 10.1016/J.ACA.2012.03.060 [retrieved on 2012-04-11] * the whole document * ----- | 1-15 | |
| Y | EP 3 872 485 A1 (UNIV ROVIRA I VIRGILI [ES]) 1 September 2021 (2021-09-01) * paragraph [0023] - paragraph [0027] * ----- | 4,10 | |

TECHNICAL FIELDS SEARCHED (IPC)

The present search report has been drawn up for all claims

2

| Place of search | Date of completion of the search | Examiner |
|---|---|---|
| Munich | 2 October 2024 | Knoll, Stephan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

## ANNEX TO THE EUROPEAN SEARCH REPORT ON EUROPEAN PATENT APPLICATION NO. EP 24 38 2358

This annex lists the patent family members relating to the patent documents cited in the above-mentioned European search report. The members are as contained in the European Patent Office EDP file on The European Patent Office is in no way liable for these particulars which are merely given for the purpose of information.

02-10-2024

| Patent document cited in search report | Publication date | Patent family member(s) | Publication date |
|---|---|---|---|
| WO 2021050867 A1 | 18-03-2021 | US 2022326171 A1 | 13-10-2022 |
| | | WO 2021050867 A1 | 18-03-2021 |
| EP 3872485 A1 | 01-09-2021 | AU 2021227237 A1 | 29-09-2022 |
| | | CA 3170527 A1 | 02-09-2021 |
| | | CN 115516299 A | 23-12-2022 |
| | | EP 3872485 A1 | 01-09-2021 |
| | | EP 4111188 A1 | 04-01-2023 |
| | | JP 2023514740 A | 07-04-2023 |
| | | US 2023085772 A1 | 23-03-2023 |
| | | WO 2021170313 A1 | 02-09-2021 |

EPO FORM P0459

For more details about this annex : see Official Journal of the European Patent Office, No. 12/82